(19) Europäisches Patentamt
European Patent Office
Office européen des brevets

(11) EP 4 778 578 A2

(12) EUROPEAN PATENT APPLICATION

(43) Date of publication:
**22.07.2026 Bulletin 2026/30**

(21) Application number: **26176707.3**

(22) Date of filing: **31.08.2023**

(51) International Patent Classification (IPC):
***A61P 13/12*** *(2006.01)*

(52) Cooperative Patent Classification (CPC):
**A61K 31/538; A61K 9/1611; A61K 9/1623; A61K 9/1635; A61K 9/1652; A61K 9/1676; A61K 9/4825; A61K 9/4858; A61K 31/70; A61P 9/04; A61P 13/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**MA TN**

(30) Priority: **01.09.2022 US 202263374257 P**
**22.08.2023 US 202363520952 P**

(62) Document number(s) of the earlier application(s) in accordance with Art. 76 EPC:
**23768350.3 / 4 580 637**

(71) Applicant: **Astrazeneca AB**
**151 85 Södertälje (SE)**

(72) Inventors:
- **KARLSSON, Eva**
**Södertälje (SE)**
- **KARLSSON, Christer**
**Södertälje (SE)**
- **HJÄRTSTAM, Johan**
**Södertälje (SE)**

(74) Representative: **AstraZeneca Intellectual Property**
**Eastbrook House**
**Shaftesbury Road**
**Cambridge CB2 8BF (GB)**

Remarks:
This application was filed on 05-05-2026 as a divisional application to the application mentioned under INID code 62.

# (54) COMBINATION OF SGLT2 INHIBITORS AND MINERALCORTICOID RECEPTOR MODULATORS

(57) The present disclosure provides for pharmaceutical compositions and oral dosage forms for treating heart failure and/or chronic kidney disease in a subject in need thereof.



Processed by Luminess, 75001 PARIS (FR)

## Description

### FIELD

The present disclosure provides a pharmaceutical composition comprising (a) one or more of a first pellet comprising (i) a first core, and (ii) a first coating on the first core, wherein the first coating comprises a mineralocorticoid receptor (MR) modulator and a first binder; and (b) one or more of a second pellet comprising (i) a second core, and (ii) a second coating on the second core, wherein the second coating comprises an SGLT2 inhibitor, wherein the SGLT2 inhibitor is about 5% to about 20% by weight of the second pellet, wherein the composition comprises about 20% to about 50% by weight of the mineralocorticoid receptor (MR) modulator; and about 1% to about 10% by weight of the SGLT2 inhibitor. The present disclosure further provides an oral dosage form comprising the pharmaceutical composition. Also provided is a method of treating heart failure and/or chronic kidney disease in a subject in need thereof, comprising administering the pharmaceutical composition or oral dosage form described herein to the subject. The present disclosure also relates to pharmaceutical compositions comprising an MR modulator and an SGLT2 inhibitor for treating heart failure and/or chronic kidney disease in a subject in need thereof.

### BACKGROUND

Chronic heart failure (HF) is a major cause of mortality, hospitalizations, and suboptimal quality of life. Even with the best possible treatment, the 5-year survival rate for HF patients is worse than for most cancers. Moreover, the prevalence of chronic HF continues to increase globally. An estimated 38 million people are affected worldwide, with over one million hospitalizations annually in both the United States and Europe. About 20% to 67% of patients with HF also have chronic kidney disease (CKD), which results in a 25% to 35% increased risk of mortality compared to patients with HF alone. See, e.g., Braunwald et al., Lancet 385(9970):812-24, 2015; Ambrosy et al., Curr Heart Fail Rep. 11(4): 416-427, 2014; Sarraf et al., Clin JAm Soc Nephrol. 4(12): 2013-26, 2009; and Ather et al., J Am Coll Cardiol. 59(11): 998-1005 (2012).

### SUMMARY

In some embodiments, the present disclosure provides a pharmaceutical composition comprising: (a) one or more of a first pellet comprising (i) a first core, and (ii) a first coating on the first core, wherein the first coating comprises a mineralocorticoid receptor (MR) modulator and a first binder; and (b) one or more of a second pellet comprising (i) a second core, and (ii) a second coating on the second core, wherein the second coating comprises an SGLT2 inhibitor, wherein the SGLT2 inhibitor is about 5% to about 20% by weight of the second pellet, wherein the composition comprises about 20% to about 50% by weight of the mineralocorticoid receptor (MR) modulator; and about 1% to about 10% by weight of the SGLT2 inhibitor.

In some embodiments, the MR modulator comprises a compound of Formula I:

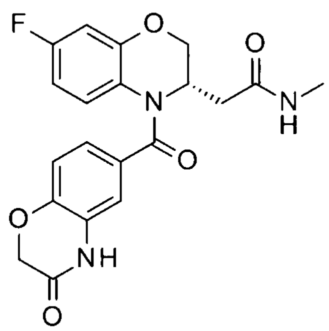

Formula I (AZD9977 or balcinrenone).

In some embodiments, the composition comprises about 20% to about 50% by weight of the MR modulator. In some embodiments, the composition comprises about 25% or about 45% by weight of the MR modulator.

**In** some embodiments, the first binder may be chosen from, by way of non-limiting example, povidone, polyethylene glycol, polyethylene oxide, hydroxypropyl methylcellulose (e.g., Hypromellose), methylcellulose, hydroxypropyl cellulose, hydroxyethyl cellulose, gelatin, starch (e.g., maize, potato, and rice), pregelatinized starch, and combinations thereof.

In some embodiments, the first binder comprises povidone. In some embodiments, the composition comprises about 1% to about 10% by weight povidone. In some embodiments, the composition comprises about 4% to about 5% by

weight povidone. In some embodiments, the composition comprises about 6% to about 7% by weight povidone.

In some embodiments, the first binder comprises povidone and hypromellose. In some embodiments, the composition comprises about 4% to about 6% by weight povidone. In some embodiments, the composition comprises about 0.5% to about 3% by weight hypromellose. In some embodiments, the composition comprises about 5% by weight povidone and about 1% by weight hypromellose.

In some embodiments, the first coating may further comprise a first lubricant. The first lubricant can be chosen, by way of non-limiting example, from sodium stearyl fumarate, magnesium stearate, stearic acid, calcium stearate, stearyl alcohol, talc, silica, or combinations thereof. In some embodiments, the first lubricant comprises sodium stearyl fumarate. In some embodiments, the first lubricant is 0% to about 0.5% by weight of the composition. In some embodiments, the first core comprises microcrystalline cellulose. In some embodiments, the first core is about 10% to about 30% by weight of the composition.

In some embodiments, the at least one SGLT2 inhibitor is chosen from compounds of Formula II:

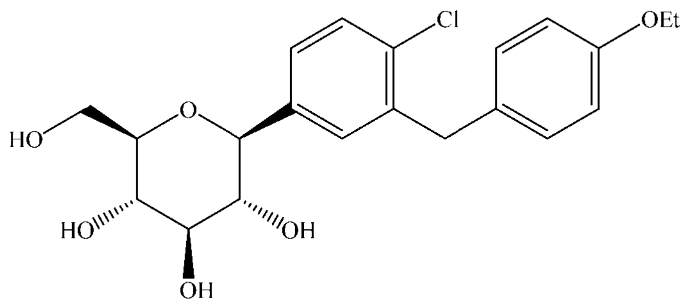

Formula II (dapagliflozin)

In some embodiments, the at least one SGLT2 inhibitor chosen from compounds of Formula II is in the form of a pharmaceutically acceptable solvate, mixed solvate, or complex. In some embodiments, the at least one compound is in the form of a non-crystalline solid (e.g., amorphous form). In some embodiments, the at least one compound is in the form of a crystalline solid.

In some embodiments, the at least one compound is in the form of a (S)-propylene glycol ((S)-PG) solvate which has the structure shown below:

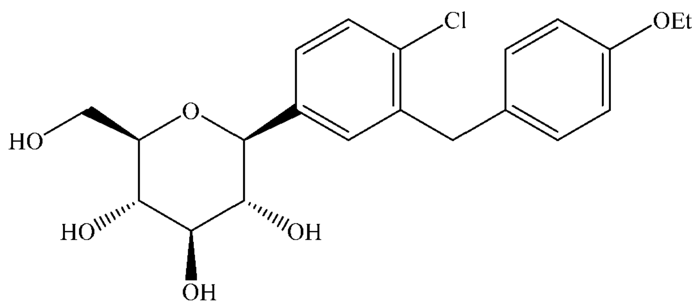

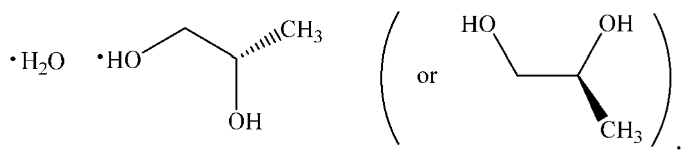

In some embodiments, the at least one compound is in the form of a crystalline S-PG solvate. Methods for preparing a (S)-PG solvate of dapagliflozin, including a crystalline S-PG solvate, are provided in U.S. Pat. No. 7,919,598.

In some embodiments, the composition comprises about 1% to about 10% by weight of the SGLT2 inhibitor. In some embodiments, the composition comprises about 2.5% to about 4% of the SGLT2 inhibitor.

In some embodiments, the SGLT2 inhibitor is about 5% to about 20% by weight of the second pellet. In some embodiments, the SGLT2 inhibitor is about 5% to about 15% by weight of the second pellet. In some embodiments, the SGLT2 inhibitor is about 10% to about 15% by weight of the second pellet. In some embodiments, the SGLT2 inhibitor is about 12% to about 14% by weight of the second pellet.

In some embodiments, the second coating further comprises a second binder, an anti-tacking agent, and optionally a second lubricant. In some embodiments, the second binder may be chosen from, for example, povidone (polyvinylpyrrolidone PVP), polyethylene glycol (PEG), polyethylene oxide (PEO), xanthan gum, cellulose derivatives such as, methylcellulose (MC), hydroxyethylcellulose (HEC), hydroxypropylcellulose (HPC), hydroxypropyl methylcellulose (HPMC), gelatin, starch (such as maize, potato or rise), pregelatinized starch, and related materials or combinations thereof. In at least one embodiment, the second binder comprises hydroxypropyl cellulose. In some embodiments, the second binder is about 0.1% to about 5% by weight of the composition. In some embodiments, the anti-tacking agent comprises talc. In some embodiments, the anti-tacking agent is about 1% to about 20% by weight of the composition. In some embodiments, the second lubricant comprises sodium stearyl fumarate. In some embodiments, the second lubricant is 0% to about 1% by weight of the composition. In some embodiments, the second core comprises a sugar, a starch, or combination thereof. In some embodiments, the second core is about 10% to about 40% by weight of the composition.

In some embodiments, the present disclosure provides a pharmaceutical composition in the form of a capsule, wherein components in the capsule may have different or the same weight percentages as in the pharmaceutical composition. In at least one embodiment, the weight percentages in the pharmaceutical composition are different than in the overall capsule. In some embodiments, the present disclosure provides a pharmaceutical composition in the form of a capsule comprising: (a) one or more of a first pellet comprising: (i) a first core comprising a microcrystalline cellulose core, wherein the first core is about 5% to about 25% by weight of the capsule; (ii) a first coating comprising (A) an MR modulator, wherein the MR modulator is AZD9977, and wherein the AZD9977 is about 10% to about 45% by weight of the capsule; (B) povidone, wherein the povidone is about 1% to about 10% by weight of the capsule; and (C) sodium stearyl fumarate, wherein the sodium stearyl fumarate is about 0.01% to about 1% by weight of the capsule; and (b) one or more of a second pellet comprising: (i) a second core comprising a sugar sphere, wherein the sugar sphere is about 5% to about 30% by weight of the capsule; (ii) a second coating comprising (A) an SGLT2 inhibitor, wherein the SGLT2 inhibitor is dapagliflozin propanediol, and wherein the dapagliflozin propanediol is about 1% to about 10% by weight of the capsule and about 5% to about 20% by weight of the second pellet; (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 0.1% by weight of the capsule.

In some embodiments, the present disclosure provides a capsule comprising about 50 mg to about 150 mg AZD9977. In some embodiments, the present disclosure provides a capsule comprises about 1 mg to about 15 mg dapagliflozin propanediol.

In some embodiments, the present disclosure provides a capsule comprising about 10 mg to about 50 mg AZD9977. In some embodiments, the present disclosure provides a capsule comprising about 10 mg to about 45 mg AZD9977. In some embodiments, the present disclosure provides a capsule comprising about 10 mg to about 40 mg AZD9977. In some embodiments, the present disclosure provides a capsule comprises about 1 mg to about 15 mg dapagliflozin propanediol. In some embodiments, the present disclosure provides a capsule comprises about 5 mg to about 10 mg dapagliflozin propanediol.

In some embodiments, the present disclosure provides an oral dosage form comprising the pharmaceutical composition described herein. In some embodiments, the oral dosage form is a capsule. In some embodiments, the pharmaceutical composition in the dosage form comprises about 50 mg AZD9977. In some embodiments, the pharmaceutical composition in the dosage form comprises about 150 mg AZD9977. In some embodiments, the pharmaceutical composition in the dosage form comprises about 10 mg dapagliflozin propanediol.

In some embodiments, the present disclosure provides an oral dosage form comprising the pharmaceutical composition described herein. In some embodiments, the oral dosage form is a capsule. In some embodiments, the pharmaceutical composition in the dosage form comprises about 10 mg to about 50 mg AZD9977. In some embodiments, the pharmaceutical composition in the dosage form comprises about 10 mg AZD9977. In some embodiments, the pharmaceutical composition in the dosage form comprises about 15 mg AZD9977. In some embodiments, the pharmaceutical composition in the dosage form comprises about 20 mg AZD9977. In some embodiments, the pharmaceutical composition in the dosage form comprises about 25 mg AZD9977. In some embodiments, the pharmaceutical composition in the dosage form comprises about 30 mg AZD9977. In some embodiments, the pharmaceutical composition in the dosage form comprises about 35 mg AZD9977. In some embodiments, the pharmaceutical composition in the dosage form comprises about 40 mg AZD9977. In some embodiments, the pharmaceutical composition in the dosage form comprises about 45 mg AZD9977. In some embodiments, the pharmaceutical composition in the dosage form comprises about 50 mg AZD9977. In some embodiments, the pharmaceutical composition in the dosage form comprises about 10 mg dapagliflozin propanediol.

In some embodiments, at least 75-85% of the MR modulator is released from the pharmaceutical composition or the oral dosage form described herein within a time range of about 15-30 minutes. In other embodiments, at least 75-85% of the SGLT2 inhibitor is released from the pharmaceutical composition or the oral dosage form described herein within a time range of about 15-30 minutes. Dissolution profiles may be tested with dissolution techniques known to those skilled in

the art, for example, methods such as USP 1 at 100 rpm or USP 2 at 70 rpm.

In some embodiments, the present disclosure provides a method of treating heart failure in a subject in need thereof, comprising administering the pharmaceutical composition or the oral dosage form described herein. Also disclosed are methods of treating chronic kidney disease in a subject in need thereof, comprising administering the pharmaceutical composition or the oral dosage form described herein.

Also disclosed are methods of treating heart failure and/or chronic kidney disease in a subject in need thereof, comprising administering the pharmaceutical composition or the oral dose form described herein.

Also disclosed are methods of treating heart failure and/or chronic kidney disease in a subject in need thereof without an associated clinically significant increase in hyperkalemia in the subject, comprising administering the pharmaceutical composition or the oral dose form described herein.

Also disclosed are methods of treating heart failure and/or chronic kidney disease in a subject in need thereof without an associated clinically significant increase in hypotension in the subject, comprising administering the pharmaceutical composition or the oral dose form described herein.

Also disclosed are methods of treating heart failure and/or chronic kidney disease in a subject in need thereof without an associated clinically significant increase in acute kidney injury risk in the subject, comprising administering the pharmaceutical composition or the oral dose form described herein.

Also disclosed are methods of treating heart failure and/or chronic kidney disease in a subject in need thereof without an associated clinically significant increase in gynecomastia risk in the subject, comprising administering the pharmaceutical composition or the oral dose form described herein.

In some embodiments, a daily dose of about 50 mg or about 150 mg of the MR modulator and a daily dose of about 10 mg of the SGLT2 inhibitor are administered. In some embodiments, the administering is once daily. In some embodiments, the pharmaceutical composition or oral dosage form is administered to the subject in a fasted state. In some embodiments, an $AUC_{last}$ and $AUC_{inf}$ of the subject following the administering are within 10% of an $AUC_{last}$ and $AUC_{inf}$ of the subject administered with separate dosage forms of the MR modulator and the SGLT2 inhibitor.

In some embodiments, a daily dose in the range of about 15 mg to about 40 mg of the MR modulator and a daily dose of about 10 mg of the SGLT2 inhibitor are administered. In some embodiments, the administering is once daily. In some embodiments, the pharmaceutical composition or oral dosage form is administered to the subject in a fasted state. In some embodiments, an $AUC_{last}$ and $AUC_{int}$ of the subject following the administering are within 10% of an $AUC_{last}$ and $AUC_{inf}$ of the subject administered with separate dosage forms of the MR modulator and the SGLT2 inhibitor.

In some embodiments, a daily dose in the range of about 15 mg to about 40 mg of AZD9977 and a daily dose of about 10 mg of the SGLT2 inhibitor are administered. In some embodiments, the administering is once daily. In some embodiments, the pharmaceutical composition or oral dosage form is administered to the subject in a fasted state. In some embodiments, an $AUC_{last}$ and $AUC_{inf}$ of the subject following the administering are within 10% of an $AUC_{last}$ and $AUC_{inf}$ of the subject administered with separate dosage forms of the MR modulator and the SGLT2 inhibitor.

## BRIEF DESCRIPTION OF DRAWINGS

The following drawings form part of the present specification and are included to further demonstrate exemplary embodiments of certain aspects of the present disclosure.

FIGS. 1A-1C show results of an in-vitro dissolution study of fixed dose combination (FDC) capsules comprising AZD9977 and dapagliflozin, as described in embodiments herein. FIG. 1A shows the in-vitro dissolution profile of dapagliflozin as tested in USP apparatus 1 at 100 rpm. FIG. 1B shows the in-vitro dissolution profile of AZD9977 as tested in USP apparatus 2 at 100 rpm. FIG. 1c shows the in-vitro dissolution profile of AZD9977 as tested in USP apparatus 1 at 100 rpm.

FIGS. 2A and 2B show exemplary pharmaceutical compositions and components thereof as described in embodiments herein. FIG. 2A shows single active dosage forms and pellets of AZD9977 and dapagliflozin. FIG. 2B shows a schematic of a clinical study to evaluate the bioavailability of the FDC capsules as compared to single active dosage forms, as described in Example 3.

FIGS. 3A and 3B show the mean plasma AZD9977 concentration versus time curves of subjects in the clinical study as described in Example 3. FIG. 3A shows the results in linear scale; FIG. 3B shows the results in semi logarithmic scale.

FIG. 4A shows a statistical comparison of pharmacokinetic parameters of AZD9977 in Group 1 (Fasted) of the clinical study described in Example 3.

FIG. 4B shows a statistical comparison of pharmacokinetic parameters of AZD9977 in Group 1 (Fed/Fasted) of the

clinical study described in Example 3.

FIG. 5A shows a statistical comparison of pharmacokinetic parameters of AZD9977 in Group 2 of the clinical study described in Example 3.

FIG. 5B shows a statistical comparison of pharmacokinetic parameters of AZD9977 for the same variant of AZD9977 provided in different capsules under fasted condition across Groups of the clinical study described in Example 3.

FIGS. 6A and 6B show the geometric mean plasma dapagliflozin concentration versus time curves of subjects in the clinical study as described in Example 3. FIG. 6A shows the results in linear scale; FIG. 6B shows the results in semi logarithmic scale.

FIG. 7A shows a statistical comparison of pharmacokinetic parameters of dapagliflozin in Group 1 (Fasted) of the clinical study described in Example 3.

FIG. 7B shows a statistical comparison of pharmacokinetic parameters of dapagliflozin in Group 1 (Fed/Fasted) of the clinical study described in Example 3.

FIG. 8A shows a statistical comparison of pharmacokinetic parameters of dapagliflozin in Group 2 of the clinical study described in Example 3.

FIG. 8B shows a statistical comparison of pharmacokinetic parameters of dapagliflozin for the same variant of dapagliflozin provided in different capsules under fasted condition across Groups of the clinical study described in Example 3.

## DETAILED DESCRIPTION

Unless otherwise defined herein, scientific and technical terms used in the present disclosure shall have the meanings that are commonly understood by one of ordinary skill in the art. Further, unless otherwise required by context, singular terms shall include pluralities and plural terms shall include the singular.

As used herein, "about" can mean plus or minus 10% of the provided value. Where ranges are provided, they are inclusive of the boundary values. "About" can additionally or alternately mean either within 10% of the stated value, or within 5% of the stated value, or in some cases within 2.5% of the stated value; or, "about" can mean rounded to the nearest significant digit.

As used herein, "between" is a range inclusive of the ends of the range. For example, a number between *x* and *y* explicitly includes the numbers x and y and any numbers that fall within x and y.

Mineralocorticoid receptor (MR) is a ligand activated transcription binding factor belonging to the oxysteroid nuclear hormone receptor class. MR activation by aldosterone in kidney tubules and epithelial cells plays a pivotal role in blood pressure control. MR activation in non-epithelial tissues promotes target organ dysfunction by stimulating inflammation, oxidative stress, and fibrosis. Further, pathological activation of MR leads to an increased risk for cardiovascular events. See, e.g., Bamberg et al., *PloS One* 13(2):e0193380, 2018. As used herein, a "MR modulator" refers to a compound that is capable of reducing MR activation, e.g., by blocking and/or preventing binding of MR to an agonist. In some embodiments, the MR modulator of the pharmaceutical composition described herein comprises a MR antagonist. Non-limiting examples of MR antagonists include spironolactone, eplerenone, canrenone, progesterone, drospirenone, gestodene, and benidipine. In some embodiments, the MR antagonist antagonizes MR in epithelial and non-epithelial tissues. In some embodiments, the MR modulator of the pharmaceutical composition described herein comprises a selective MR modulator. In some embodiments, the selective MR modulator is capable of antagonizing MR in non-epithelial cells but do not substantially affect MR activation in epithelial cells. Non-limiting examples of selective MR modulators include finerenone and AZD9977. AZD9977, or balcinrenone, is further described in, e.g., WO 2016/001631 and is a compound of Formula I:

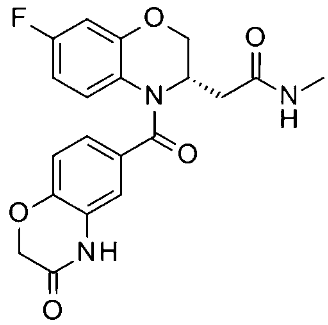

Formula I (AZD9977 or balcinrenone).

Sodium-glucose cotransporter-2 (SGLT2) is the major cotransporter involved in glucose reabsorption in the kidney. As used herein, "SGLT2 inhibitor," also known as gliflozins or flozins, inhibit SGLT2 activity. In some embodiments, SGLT2 inhibitors inhibit reabsorption of glucose in the kidney and therefore lower blood sugar. SGLT2 inhibitors can slow the progression of kidney disease, reduce HF, and lower the risk of kidney failure and death in subjects with CKD and with kidney disease and type 2 diabetes. See, e.g., "SGLT2 Inhibitors," National Kidney Foundation (<kidney.org/atoz/content/sglt2-inhibitors>; accessed June 2022). In some embodiments, the SGLT2 inhibitor of the pharmaceutical composition described herein comprises canagliflozin, dapagliflozin, empagliflozin, ertugliflozin, or combination thereof. In some embodiments, the SGLT2 inhibitor comprises a solvate form of canagliflozin, dapagliflozin, empagliflozin, and/or ertugliflozin. In some embodiments, the SGLT2 inhibitor is in a non-crystalline form (e.g., amorphous form) or crystalline form. In some embodiments, the SGLT2 inhibitor comprises dapagliflozin propanediol. In some embodiments, "dapagliflozin propanediol" refers to a solvate form of dapagliflozin comprising a 1:1:1 ratio of dapagliflozin, (*S*)-(+)-1,2-propanediol, and water. In some embodiments, the theoretical amount of dapagliflozin as the non-solvated form is about 81.29% (w/w) of the dapagliflozin propanediol.

In some embodiments, the present disclosure provides a combination of an MR modulator and SGLT2 inhibitor in a single dosage form, also known as a "fixed dose combination" or FDC formulation, which is useful for the treatment of HF and/or CKD. In some embodiments, an FDC formulation comprising the MR modulator and SGLT2 inhibitor provides a greater therapeutic benefit as compared to each active ingredient alone. In some embodiments, administration of an FDC formulation comprising the MR modulator and SGLT2 inhibitor provides a greater therapeutic benefit as compared to co-administration of the MR modulator and SGLT2 inhibitor in separate dosage forms containing a single active ingredient.

Challenges of developing an FDC formulation include, for example, ensuring the active ingredients in the FDC formulation have physicochemical compatibility; preventing undesirable mechanical powder flow and compression characteristics of a multi-active ingredient blend; and providing a reasonable dosage form size.

In some embodiments, the FDC formulation provided herein comprises two active ingredients belonging to different categories according to the Biopharmaceutics Classification System (BCS), e.g., AZD9977 and dapagliflozin. AZD9977 is a class 4 drug, characterized by low solubility and low permeability. Dapagliflozin is a BCS class 3 drug, characterized by high solubility, rapid dissolution, and low permeability. Thus, a particular challenge of developing an FDC formulation comprising AZD9977 and dapagliflozin is ensuring that the active ingredients have comparable in-vitro dissolution and in-vivo bioavailability as compared to those observed for the single active ingredient formulation. Further, a high drug load for AZD9977 (e.g., about 50 to about 200 mg, or about 120 to about 180 mg in the dosage form, or about 140 to about 160 mg, or about 50 mg or 150 mg in the dosage form) is desired, yet the dosage form size should be maintained at a size that does not hinder patient compliance. In some embodiments, the FDC formulation is provided, for example, in a size 0, size 1 such as size 1E hard gelatin capsule.

To address such challenges, provided herein is a composition comprising the first active ingredient, e.g., MR modulator, in a first pellet; and the second active ingredient, e.g., SGLT2 inhibitor, in a second pellet. In some embodiments, the first and second pellets that respectively comprise the first and second active ingredients, e.g., the MR modulator and SGLT2 inhibitor, each comprises a core and a coating coated on the core. By adjusting the amounts and type of components in each coating according to the physicochemical properties, e.g., solubility, reactivity, and/or stability of the active ingredient therein, the desired drug load and dissolution profile for each active ingredient was achieved.

In some embodiments, the present disclosure provides a pharmaceutical composition comprising: (a) one or more of a first pellet comprising (i) a first core, and (ii) a first coating on the first core, wherein the first coating comprises a mineralocorticoid receptor (MR) modulator and a first binder comprising povidone; and (b) one or more of a second pellet comprising (i) a second core, and (ii) a second coating on the second core, wherein the second coating comprises an SGLT2 inhibitor, wherein the SGLT2 inhibitor is about 5% to about 20% by weight of the second pellet; wherein the composition comprises about 20% to about 50% by weight of the mineralocorticoid receptor (MR) modulator; and about 1% to about 10% by weight of the SGLT2 inhibitor. In some embodiments, the present disclosure provides an oral dosage form comprising the pharmaceutical composition described herein. In some embodiments, the oral dosage form is a

capsule, caplet or a tablet. In some embodiments, the oral dosage form is a capsule. In some embodiments, the capsule is a size 000, 00, 00EL, 0, 1, 2 or 3 gelatin capsule. In some embodiments, the capsule is a size 0 gelatin capsule. In some embodiments, the capsule is a size 1 gelatin capsule, such as a 1E gelatin capsule.

**FIRST PELLET**

In some embodiments, the first pellet of the composition comprises a first core and a first coating on the first core. In some embodiments, the first coating comprises an MR modulator and a first binder. In some embodiments, the first coating comprises an MR modulator, a first binder, and a lubricant.

In some embodiments, the first coating comprises an MR modulator. Exemplary MR modulators are provided herein. In some embodiments, the MR modulator comprises an MR antagonist, a selective MR modulator, or combination thereof. In some embodiments, the MR modulator comprises spironolactone, eplerenone, canrenone, progesterone, drospirenone, gestodene, benidipine, finerenone, AZD9977, or combination thereof. In some embodiments, the MR modulator comprises AZD9977 (balcinrenone).

In some embodiments, MR modulator is about 20% to about 60%, or about 25% to about 55%, or about 20% to about 50%, or about 30% to about 50%, or about 35% to about 48%, or about 40% to about 45% by weight of the composition. In some embodiments, the MR modulator is about 35%, about 36%, about 37%, about 38%, about 39%, about 40%, about 41%, about 42%, about 43%, about 44%, about 45% about 46%, about 47%, about 48%, about 49%, or about 50% by weight of the composition.

In some embodiments, the MR modulator comprises AZD9977. In some embodiments, the composition comprises about 20% to about 60% by weight AZD9977. In some embodiments, the composition comprises about 25% to about 55% by weight AZD9977. In some embodiments, the composition comprises about 20% to about 50% by weight AZD9977. In some embodiments, the composition comprises about 25% to about 45% by weight AZD9977. In some embodiments, the composition comprises about 40% to about 45% by weight AZD9977.In some embodiments, the composition comprises about 35%, about 36%, about 37%, about 38%, about 39%, about 40%, about 41%, about 42%, about 43%, about 44%, about 45%, about 46%, about 47%, about 48%, about 49%, or about 50% by weight AZD9977. In some embodiments, the composition comprises about 28% by weight AZD9977. In some embodiments, the composition comprises about 43% by weight AZD9977.

In some embodiments, the first coating comprises a first binder. As used herein, a "binder" refers to an excipient that holds together the components of a composition, e.g., powders, granules, and other dry ingredients. Non-limiting examples of binders that may be used in the compositions described herein include gelatin; cellulose and derivatives thereof, e.g., methylcellulose, microcrystalline cellulose, hydroxypropyl methylcellulose (also referred to herein as "hypromellose" or "HPMC"), hydroxypropyl cellulose, hydroxyethyl cellulose, sodium croscarmellose; polyvinylpyrrolidone (also referred to herein as "povidone" or "PVP"); starches (e.g., maize, potato, and rice); pregelatinized starch, sugars such as sucrose, lactose, and derivatives thereof; sugar alcohols such as mannitol; sweeteners such as isomalt; and polyethylene glycol (PEG), polyethylene oxide (PEO) and combinations thereof.

In some embodiments, the first binder is about 1% to about 20%, or about 2% to about 15%, or about 3% to about 10%, or about 4% to about 8%, or about 6% to about 7% by weight of the composition. In some embodiments, the first binder is about 1%, about 2%, about 3%, about 4%, about 5%, about 6%, about 7%, about 8%, about 9%, or about 10% by weight of the composition. In some embodiments, the first binder is about 4.2%, about 6%, about 6.32%, or about 6.5% by weight of the composition. In some embodiments, the first binder comprises povidone, hypromellose, or combination thereof.

In some embodiments, the first binder comprises povidone. In some embodiments, the composition comprises about 1% to about 20% by weight povidone. In some embodiments, the composition comprises about 2% to about 15% by weight povidone. In some embodiments, the composition comprises about 3% to about 10% by weight povidone. In some embodiments, the composition comprises about 4% to about 8% by weight povidone. In some embodiments, the composition comprises about 6% to about 7% by weight povidone. In some embodiments, the composition comprises about 1%, about 2%, about 3%, about 4%, about 5%, about 6%, about 7%, about 8%, about 9%, or about 10% by weight povidone. In some embodiments, the composition comprises about 6.0%, about 6.1%, about 6.2%, about 6.3%, about 6.4%, about 6.5%, about 6.6%, about 6.7%, about 6.8%, about 6.9%, or about 7% by weight povidone. In some embodiments, the composition comprises about 4.2% by weight povidone. In some embodiments, the composition comprises about 6.5% by weight povidone.

In some embodiments, the first binder comprises povidone and hypromellose. In some embodiments, the composition comprises about 1% to about 10% by weight povidone, or about 2% to about 8% by weight povidone, or about 3% to about 7% by weight povidone, or about 4% to about 6% by weight povidone. In some embodiments, the composition comprises about 4.50%, about 4.55%, about 4.60%, about 4.65%, about 4.70%, about 4.75%, about 4.80%, about 4.85%, about 4.90%, about 4.95% about 5.00%, about 5.05%, about 5.10%, about 5.15%, about 5.20%, about 5.25%, about 5.30%, about 5.35%, about 5.40%, about 5.45%, or about 5.50% povidone. In some embodiments, the composition

comprises about 0.1% to about 5% by weight hypromellose, or about 0.5% to about 3% by weight hypromellose, or about 0.7% to about 2% by weight hypromellose, or about 0.8% to about 1% by weight hypromellose.

In some embodiments, the composition comprises about 1% to about 10% by weight povidone, or about 2% to about 8% by weight povidone, or about 3% to about 7% by weight povidone, or about 5% by weight povidone; and about 1% by weight hypromellose. In some embodiments, the composition comprises about 5% by weight povidone; and about 0.1% to about 10% by weight hypromellose, or about 0.5% to about 3% by weight hypromellose, or about 0.7% to about 2% by weight hypromellose, or about 0.8% to about 1% by weight hypromellose. In some embodiments, the composition comprises about 5% povidone and about 1% hypromellose. In some embodiments, the composition comprises about 5.35% povidone and about 0.97% hypromellose. In some embodiments, the composition comprises about 4.7% povidone and about 0.85% hypromellose.

In some embodiments, the first coating further comprises a first lubricant, a solvent, or combination thereof. In some embodiments, the solvent comprises water. In some embodiments, the solvent is present prior to coating the first coating onto the first core, and removed (e.g., evaporated) when the first coating is coated onto the first core. As used herein, a "lubricant" is a pharmaceutical excipient that can reduce friction between other components and inhibit particle agglomeration. Non-limiting examples of lubricants include sodium stearyl fumarate, hydroxypropyl cellulose, hydrogenated vegetable oil, silicon dioxide, stearic acid, magnesium stearate, calcium stearate, stearyl alcohol, talc, silica, glyceride esters such as glyceryl monostearate, glyceryl tribehenate, and glyceryl dibehenate, and sugar esters such as sorbitan monostearate and sucrose monopalmitate, and combinations thereof.

In some embodiments, the first lubricant comprises sodium stearyl fumarate. In some embodiments, the first lubricant is 0% to about 1%, or about 0.01% to about 0.8%, or about 0.01% to about 0.5%, or about 0.1% to about 0.4%, or about 0.3% to about 0.35% by weight of the composition. In some embodiments, the composition comprises about 0.1%, about 0.15%, about 0.2%, about 0.25%, about 0.3%, about 0.35%, about 0.4%, about 0.45%, about 0.5% by weight sodium stearyl fumarate. In some embodiments, the composition comprises about 0.1% by weight sodium stearyl fumarate. In some embodiments, the composition comprises about 0.15% by weight sodium stearyl fumarate.

In some embodiments, the composition comprises a first pellet, wherein the first pellet comprises a first coating comprising:

about 20% to about 50% by weight an MR modulator;
about 1% to about 10% by weight a first binder; and
optionally, about 0.01% to about 0.5% by weight a first lubricant,

wherein all weights are by weight of the composition. In some embodiments, the MR modulator comprises AZD9977.

In some embodiments, the first coating described herein is coated on a first core. In some embodiments, the first core is pharmaceutically inert, i.e., does not substantially react with any other component in the composition described herein. In some embodiments, the first core may be chosen from, for example, microcrystalline cellulose, sucrose, isomalt, calcium phosphate (e.g., anhydrous dibasic calcium phosphate), tartaric acid, silica, xylitol, mannitol, lactose, calcium carbonate, starch, silicone dioxide, non-pareil core, or combinations thereof.

In some embodiments, the first core is about 10% to about 50%, or about 10% to about 40%, or about 10% to about 30%, or about 15% to about 30%, or about 15% to about 25%, or about 20% to about 24% by weight of the composition. In some embodiments, the first core is about 15%, about 16%, about 17%, about 18%, about 19%, or about 20%, about 21%, about 22%, about 23%, about 24%, or about 25% by weight of the composition. In some embodiments, the first core comprises microcrystalline cellulose. In some embodiments, the composition comprises about 14.4%, about 21.1%, or about 22.3% by weight microcrystalline cellulose.

In some embodiments, the composition comprises a first pellet comprising:

a first coating comprising:

about 20% to about 60%, or about 25% to about 55%, or about 30% to about 50%, or about 35% to about 48%, or about 40% to about 45% by weight MR modulator;
about 1% to about 20%, or about 2% to about 15%, or about 3% to about 10%, or about 4% to about 8%, or about 6% to about 7% by weight povidone;
optionally about 0.01% to about 0.5%, or about 0.08% to about 0.5%, or about 0.1% to about 0.5%, or about 0.2% to about 0.4%, or about 0.3% to about 0.35% by weight sodium stearyl fumarate; and

a first core comprising:

about 10% to about 50%, or about 12% to about 40%, or about 15% to about 30%, or about 15% to about 25%, or about 20% to about 24%, or about 21%, or about 23% by weight microcrystalline cellulose,

wherein all weights are by weight of the composition. In some embodiments, the MR modulator comprises AZD9977.

In some embodiments, the composition comprises a first pellet comprising:

a first coating comprising

about 20-30% by weight an MR modulator;
about 4-5% by weight povidone; and
a first core comprising about 10-15% by weight microcrystalline cellulose,

wherein all weights are by weight of the composition. In some embodiments, the MR modulator comprises AZD9977.

In some embodiments, the composition comprises a first pellet comprising:

a first coating comprising

about 40-45% by weight an MR modulator;
about 6-7% by weight povidone; and
a first core comprising about 20-25% by weight microcrystalline cellulose,

wherein all weights are by weight of the composition. In some embodiments, the MR modulator comprises AZD9977.

In some embodiments, the composition comprises a first pellet comprising:

a first coating comprising:

about 20% to about 60%, or about 25% to about 55%, or about 30% to about 50%, or about 35% to about 48%, or about 40% to about 45% by weight MR modulator;
about 1% to about 10%, or about 2% to about 8%, or about 3% to about 7%, or about 4% to about 6% by weight povidone; and

a first core comprising:

about 10% to about 50%, or about 12% to about 40%, or about 15% to about 30%, or about 15% to about 25%, or about 20% to about 24%, or about 21%, or about 23% by weight microcrystalline cellulose,

wherein all weights are by weight of the composition. In some embodiments, the MR modulator comprises AZD9977.

In some embodiments, the composition comprises a first pellet comprising:

a first coating comprising

about 28% by weight an MR modulator;
about 4.2% by weight povidone; and
a first core comprising about 14.4% by weight microcrystalline cellulose,

wherein all weights are by weight of the composition. In some embodiments, the MR modulator comprises AZD9977.

In some embodiments, the composition comprises a first pellet comprising:

a first coating comprising

about 43% by weight an MR modulator;
about 6.5% by weight povidone; and
a first core comprising about 22.3% by weight microcrystalline cellulose,

wherein all weights are by weight of the composition. In some embodiments, the MR modulator comprises AZD9977.

## SECOND PELLET

**In** some embodiments, the second pellet of the composition comprises a second core and a second coating on the second core. In some embodiments, the second coating comprises an SGLT2 inhibitor and a second binder. In some embodiments, the second coating comprises an SGLT2 inhibitor, a second binder, an anti-tacking agent, and optionally a second lubricant.

**In** some embodiments, the second coating comprises an SGLT2 inhibitor. Exemplary SGLT2 inhibitors are provided herein. In some embodiments, the SGLT2 inhibitor comprises canagliflozin, dapagliflozin, empagliflozin, ertugliflozin, or combination thereof. In some embodiments, the SGLT2 inhibitor comprises dapagliflozin or a solvate thereof. Dapagliflozin solvates are further described herein. In some embodiments, the SGLT2 inhibitor comprises dapagliflozin propanediol.

**In** some embodiments, the SGLT2 inhibitor is about 1% to about 10%, or about 2% to about 8%, or about 2.5% to about 7.5%, or about 3% to about 7% by weight of the composition. In some embodiments, the SGLT2 inhibitor is about 2%, about 2.1%, about 2.2%, about 2.3%, about 2.4%, about 2.5%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9%, about 5%, about 5.1%, about 5.2%, about 5.3%, about 5.4%, about 5.5%, about 5.6%, about 5.7%, about 5.8%, about 5.9%, about 6%, about 6.1%, about 6.2%, about 6.3%, about 6.4%, about 6.5%, about 6.6%, about 6.7%, about 6.8%, about 6.9%, or about 7% by weight of the composition.

**In** some embodiments, the SGLT2 inhibitor comprises dapagliflozin or solvate thereof. In some embodiments, the SGLT2 inhibitor comprises dapagliflozin propanediol. In some embodiments, the composition comprises about 1% to about 10% by weight dapagliflozin or solvate thereof. In some embodiments, the composition comprises about 2% to about 8% by weight dapagliflozin or solvate thereof. In some embodiments, the composition comprises about 2.5% to about 4.5% by weight dapagliflozin or solvate thereof. In some embodiments, the composition comprises about 3% to about 4% by weight dapagliflozin or solvate thereof. In some embodiments, the composition comprises is about 2%, about 2.1%, about 2.2%, about 2.3%, about 2.4%, about 2.5%, about 2.5%, about 2.6%, about 2.7%, about 2.8%, about 2.9%, about 3%, about 3.1%, about 3.2%, about 3.3%, about 3.4%, about 3.5%, about 3.6%, about 3.7%, about 3.8%, about 3.9%, about 4%, about 4.1%, about 4.2%, about 4.3%, about 4.4%, about 4.5%, about 4.6%, about 4.7%, about 4.8%, about 4.9% or about 5% by weight dapagliflozin or solvate thereof. In some embodiments, the composition comprises about 3.6% by weight dapagliflozin propanediol. In some embodiments, the composition comprises about 6.9% by weight dapagliflozin propanediol.

In some embodiments, the second coating further comprises a second binder, an anti-tacking agent, and a second lubricant. As used herein, a "lubricant" is a pharmaceutical excipient that can reduce friction between other components and inhibit particle agglomeration. Non-limiting examples of lubricants include sodium stearyl fumarate, hydroxypropyl cellulose, hydrogenated vegetable oil, silicon dioxide, stearic acid, magnesium stearate, calcium stearate, stearyl alcohol, talc, silica, glyceride esters such as glyceryl monostearate, glyceryl tribehenate, and glyceryl dibehenate, and sugar esters such as sorbitan monostearate and sucrose monopalmitate, and combinations thereof.

In some embodiments, the second coating comprises a second binder. Exemplary binders are further described herein. In some embodiments, the second binder is about 0.05% to about 10% by weight of the composition. In some embodiments, the second binder is about 0.1% to about 5% by weight of the composition. In some embodiments, the second binder is about 0.2% to about 0.8% by weight of the composition. In some embodiments, the second binder is about 0.4% to about 0.5% by weight of the composition. In some embodiments, the second binder is about 0.4%, about 0.41%, about 0.42%, about 0.43%, about 0.44%, about 0.45%, about 0.46%, about 0.47%, about 0.48%, about 0.49%, or about 0.5% by weight of the composition. In some embodiments, the second binder comprises hydroxypropyl cellulose. In some embodiments, the composition comprises about 0.5% hydroxypropyl cellulose. In some embodiments, the composition comprises about 1% hydroxypropyl cellulose.

In some embodiments, the second coating comprises an anti-tacking agent. As used herein, an "anti-tacking agent" is an excipient that prevents aggregation of particles, e.g., by absorbing moisture. Non-limiting examples of anti-tacking agents include tricalcium phosphate, magnesium trisilicate, calcium carbonate, magnesium carbonate, talc (also known as talcum powder or hydrated magnesium silicate), powdered cellulose, sodium bicarbonate, sodium silicate, calcium silicate, sodium aluminosilicate, potassium aluminum silicate, calcium aluminosilicate, bentonite, aluminum silicate, colloidal silica and polydimethylsiloxane, and combinations thereof.

In some embodiments, the anti-tacking agent comprises talc. In some embodiments, the anti-tacking agent is about 1% to about 20%, about 1% to about 15%, about 1% to about 12%, about 3% to about 10%, or about 7% to about 9% by weight of the composition. In some embodiments, the anti-tacking agent is about 7%, about 7.1%, about 7.2%, about 7.3%, about 7.4%, about 7.5%, about 7.6%, about 7.7%, about 7.8%, about 7.9%, about 8%, about 8.1%, about 8.2%, about 8.3%, about 8.4%, about 8.5%, about 8.6%, about 8.7%, about 8.8%, about 8.9%, or about 9% by weight of the composition. In some embodiments, the composition comprises about 7.8% by weight talc. In some embodiments, the

anti-tacking agent is about 14%, about 14.1%, about 14.2%, about 14.3%, about 14.4%, about 14.5%, about 14.6%, about 14.7%, about 14.8%, about 14.9%, about 15%, about 15.1%, about 15.2%, about 15.3%, about 15.4%, about 15.5%, about 15.6%, about 15.7%, about 15.8%, about 15.9%, or about 16% by weight of the composition. In some embodiments, the composition comprises about 15.1% talc.

In some embodiments, the second coating may comprise a second lubricant, a solvent, or combination thereof. In some embodiments, the solvent comprises water. In some embodiments, the solvent is present prior to coating the second coating onto the second core, and removed (e.g., evaporated) when the second coating is coated onto the second core. Exemplary lubricants are further described herein. In some embodiments, the second lubricant is 0% to about 1%, or about 0.05% to about 0.5%, or about 0.08% to about 0.3%, or about 0.1% to about 0.2% by weight of the composition. In some embodiments, the second lubricant is about 0.1%, about 0.11%, about 0.12%, about 0.13%, about 0.14%, about 0.15%, about 0.16%, about 0.17%, about 0.18%, about 0.19%, or about 0.2% by weight of the composition. In some embodiments, the second lubricant comprises sodium stearyl fumarate. In some embodiments, the composition comprises about 0.1% by weight sodium stearyl fumarate. In some embodiments, the composition comprises about 0.6% by weight sodium stearyl fumarate.

In some embodiments, the composition comprises a second pellet, wherein the second pellet comprises a second coating comprising:

about 1% to about 30% by weight an SGLT2 inhibitor;
about 0.1% to about 5% by weight a second binder;
about 1% to about 20% by weight an anti-tacking agent; and
optionally, about 0.01% to about 1% by weight a second lubricant,

wherein all weights are by weight of the composition. In some embodiments, the SGLT2 inhibitor is dapagliflozin propanediol.

In some embodiments, the composition comprises a second pellet, wherein the second pellet comprises a second coating comprising:

about 1% to about 30% by weight an SGLT2 inhibitor, wherein the SGLT2 inhibitor is about 10% to about 15%, or about 12% to about 14% by weight of the second pellet;
about 0.5% to about 1% by weight a second binder;
about 5% to about 15% by weight an anti-tacking agent; and
optionally, about 0.05% to about 0.1% by weight a second lubricant,

wherein all weights are by weight of the composition. In some embodiments, the SGLT2 inhibitor is dapagliflozin propanediol.

In some embodiments, the second coating described herein is coated on a second core. In some embodiments, the second core is pharmaceutically inert as described herein. In some embodiments, the second core comprises microcrystalline cellulose, sucrose, isomalt, calcium phosphate (e.g., anhydrous dibasic calcium phosphate), tartaric acid, silica, xylitol, mannitol, lactose, calcium carbonate, starch, silicon dioxide, or combination thereof. In some embodiments, the second core is a sugar core. Sugar cores are also known as "neutral pellet," "nonpareil core," "sugar sphere," or "sugar bead," and include a combination of a sugar, e.g., sucrose, and starch. Exemplary sugar cores include, but are not limited to, those marketed under the trade name SUGLETS®, SANAQ®, and VIVAPHARM® Sugar Spheres.

In some embodiments, the second core is about 5% to about 40%, or about 10% to about 40%, or about 10% to about 30%, or about 10% to about 22%, or about 10% to about 15%, or about 11% to about 13%, or about 20% to about 25%, or about 21% to about 23% by weight of the composition. In some embodiments, the second core is about 10%, about 11%, about 12%, about 13%, about 14%, about 15%, about 16%, about 17%, about 18%, about 19%, about 20%, about 21%, about 22%, about 23%, about 24%, about 25%, about 26%, about 27%, about 28%, about 29%, about 30%, about 31%, about 32%, about 33%, about 34%, about 35%, by weight of the composition. In some embodiments, the second core is about 15.8% by weight of the composition. In some embodiments, the second core is about 30.6% by weight of the composition. In some embodiments, the second core comprises a sugar, a starch, or combination thereof. In some embodiments, the second core is a sugar core.

In some embodiments, the composition comprises a second pellet comprising:

a second coating comprising:

about 1% to about 10%, or about 2% to about 5%, or about 2.5% to about 4.5%, or about 3% to about 4% by weight SGLT2 inhibitor, wherein the SGLT2 inhibitor is about 6% to about 15% by weight of the second pellet;
about 0.1% to about 5%, or about 0.1% to about 2.5%, or about 0.25% to about 1.5%, or about 0.5% to about 1%

by weight hydroxypropyl cellulose;
about 1% to about 20%, about 1% to about 17.5%, about 5% to about 15%, about 7% to about 10%, or about 7% to about 9% by weight talc; and
optionally about 0.01% to about 1%, or about 0.05% to about 1%, or about 0.06% to about 0.5%, or about 0.06% to about 0.1% by weight sodium stearyl fumarate; and

a second core comprising:

about 5% to about 40%, or about 10% to about 40%, or about 15% to about 30%, by weight a sugar core,

wherein all weights are by weight of the composition. In some embodiments, the SGLT2 inhibitor comprises dapagliflozin.

In some embodiments, the composition comprises a second pellet comprising:

a second coating comprising:

about 3.6% by weight an SGLT2 inhibitor, wherein the SGLT2 inhibitor is about 5% to about 15% by weight of the second pellet;
about 0.5% by weight hydroxypropyl cellulose;
about 7.8% by weight talc; and
optionally about 0.06% sodium stearyl fumarate; and

a second core comprising about 15.8% by weight a sugar core,
wherein all weights are by weight of the composition. In some embodiments, the SGLT2 inhibitor comprises dapagliflozin.

In some embodiments, the composition comprises a second pellet comprising:

a second coating comprising:

about 6.9% by weight an SGLT2 inhibitor, wherein the SGLT2 inhibitor is about 5% to about 15% by weight of the second pellet;
about 1% by weight hydroxypropyl cellulose;
about 15% by weight talc; and
optionally about 0.1% sodium stearyl fumarate; and

a second core comprising about 30.5% by weight a sugar core,
wherein all weights are by weight of the composition. In some embodiments, the SGLT2 inhibitor comprises dapagliflozin.

**COMBINATION**

In some embodiments, the composition comprising the first and second pellets as described herein is comprised in a single dosage form. In some embodiments, the composition is comprised in an oral dosage form. In some embodiments, the oral dosage form is a capsule or a tablet.
In some embodiments, the MR modulator of the pharmaceutical composition described herein comprises AZD9977, and the SGLT2 inhibitor of the pharmaceutical composition comprises dapagliflozin or solvate thereof. In some embodiments, the AZD9977 is comprised in a first pellet as described herein, and the dapagliflozin or solvate thereof is comprised in a second pellet as described herein.
In some embodiments, the present disclosure provides a pharmaceutical composition in the form of a capsule, comprising:

(a) one or more of a first pellet, comprising:

i. a first core comprising a microcrystalline cellulose core, wherein the first core is about 5% to about 25% by weight of the capsule;
ii. A first coating comprising (A) an MR modulator, wherein the MR modulator is AZD9977, and wherein the

AZD9977 is about 10% to about 45% by weight of the capsule; (B) povidone, wherein the povidone is about 1% to about 10% by weight of the capsule; and (C) sodium stearyl fumarate, wherein the sodium stearyl fumarate is about 0.01% to about 1% by weight of the capsule; and

(b) one or more of a second pellet comprising:

i. a second core comprising a sugar sphere, wherein the sugar sphere is about 5% to about 30% by weight of the capsule;
ii. A second coating comprising (A) an SGLT2 inhibitor, wherein the SGLT2 inhibitor is dapagliflozin propanediol, and wherein the dapagliflozin propanediol is about 1% to about 10% by weight of the capsule; (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 0.1% by weight of the capsule. In some embodiments, the composition is comprised in an oral dosage form. In some embodiments, the oral dosage form is a capsule.

In some embodiments, the present disclosure provides a pharmaceutical composition in the form of a capsule, comprising:

(a) one or more of a first pellet, comprising:

i. a first core comprising a microcrystalline cellulose core, wherein the first core is about 5% to about 20% by weight of the capsule;
ii. A first coating comprising (A) an MR modulator, wherein the MR modulator is AZD9977, and wherein the AZD9977 is about 15% to about 40% by weight of the capsule; (B) povidone, wherein the povidone is about 2% to about 6% by weight of the capsule; and (C) sodium stearyl fumarate, wherein the sodium stearyl fumarate is about 0.02% to about 0.4% by weight of the capsule; and

(b) one or more of a second pellet comprising:

i. a second core comprising a sugar sphere, wherein the sugar sphere is about 10% to about 22% by weight of the capsule;
ii. A second coating comprising (A) an SGLT2 inhibitor, wherein the SGLT2 inhibitor is dapagliflozin, and wherein the dapagliflozin is about 2% to about 5% by weight of the capsule; (B) hydroxypropyl cellulose, wherein the hydroxypropyl cellulose is about 0.4% to about 0.6% by weight of the capsule; (C) talc, wherein the talc is about 5% to about 12% by weight of the capsule; and (D) sodium stearyl fumarate, wherein the sodium stearyl fumarate is about 0.02% to about 0.08% by weight of the capsule. In some embodiments, the composition is comprised in an oral dosage form. In some embodiments, the oral dosage form is a capsule.

In some embodiments, the present disclosure provides a pharmaceutical composition in the form of a capsule, comprising:

(a) one or more of a first pellet, comprising:

i. a first core comprising a microcrystalline cellulose core, wherein the first core is about 18% by weight of the capsule;
ii. A first coating comprising (A) an MR modulator, wherein the MR modulator is AZD9977, and wherein the AZD9977 is about 35% by weight of the capsule; (B) povidone, wherein the povidone is about 5.3% by weight of the capsule; and (C) sodium stearyl fumarate, wherein the sodium stearyl fumarate is about 0.12% by weight of the capsule; and

(b) one or more of a second pellet comprising:

i. a second core comprising a sugar sphere, wherein the sugar sphere is about 12.8% by weight of the capsule;
ii. A second coating comprising (A) an SGLT2 inhibitor, wherein the SGLT2 inhibitor is dapagliflozin, and wherein the dapagliflozin is about 2.9% by weight of the capsule; (B) hydroxypropyl cellulose, wherein the hydroxypropyl cellulose is about 0.38% by weight of the capsule; (C) talc, wherein the talc is about 6.3% by weight of the capsule; and (D) sodium stearyl fumarate, wherein the sodium stearyl fumarate is about 0.04% by weight of the capsule. In some embodiments, the composition is comprised in an oral dosage form. In some embodiments, the oral dosage

form is a capsule.

In some embodiments, the present disclosure provides a pharmaceutical composition in the form of a capsule, comprising:

(a) one or more of a first pellet, comprising:

i. a first core comprising a microcrystalline cellulose core, wherein the first core is about 9.9% by weight of the capsule;
ii. A first coating comprising (A) an MR modulator, wherein the MR modulator is AZD9977, and wherein the AZD9977 is about 19.2% by weight of the capsule; (B) povidone, wherein the povidone is about 2.9% by weight of the capsule; and (C) sodium stearyl fumarate, wherein the sodium stearyl fumarate is about 0.06% by weight of the capsule; and

(b) one or more of a second pellet comprising:

i. a second core comprising a sugar sphere, wherein the sugar sphere is about 21% by weight of the capsule;
ii. A second coating comprising (A) an SGLT2 inhibitor, wherein the SGLT2 inhibitor is dapagliflozin, and wherein the dapagliflozin is about 4.7% by weight of the capsule; (B) hydroxypropyl cellulose, wherein the hydroxypropyl cellulose is about 0.63% by weight of the capsule; (C) talc, wherein the talc is about 10.4% by weight of the capsule; and (D) sodium stearyl fumarate, wherein the sodium stearyl fumarate is about 0.07% by weight of the capsule. In some embodiments, the composition is comprised in an oral dosage form. In some embodiments, the oral dosage form is a capsule.

In some embodiments, the present disclosure provides a pharmaceutical composition in the form of a capsule, comprising:

(a) one or more of a first pellet comprising:

i. a first core comprising a microcrystalline cellulose core, wherein the first core is about 5% to about 25% by weight of the capsule;
ii. A first coating comprising (A) an MR modulator, wherein the MR modulator is AZD9977, and wherein the AZD9977 is about 10% to about 45% by weight of the capsule, (B) povidone, wherein the povidone is about 2% to about 8% by weight of the capsule; (C) hypromellose, wherein the hypromellose is about 0.5% to about 3% by weight of the capsule; and (D) sodium stearyl fumarate, wherein the sodium stearyl fumarate is about 0.01% to about 0.5% by weight of the capsule; and

(b) one or more of a second pellet comprising:

i. a second core comprising a sugar sphere, wherein the second core is about 5% to about 30% by weight of the capsule;
ii. A second coating comprising (A) an SGLT2 inhibitor, wherein the SGLT2 inhibitor is dapagliflozin propanediol, and wherein the dapagliflozin propanediol is about 1% to about 10% by weight of the capsule and about 5% to about 20% by weight of the second pellet, (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 1% by weight of the capsule. In some embodiments, the composition is comprised in an oral dosage form. In some embodiments, the oral dosage form is a capsule.

In some embodiments, the present disclosure provides a pharmaceutical composition in the form of a capsule, comprising:

(a) one or more of a first pellet comprising:

i. a first core comprising a microcrystalline cellulose core, wherein the first core is about 23.9% by weight of the capsule;
ii. A first coating comprising (A) an MR modulator, wherein the MR modulator is AZD9977, and wherein the AZD9977 is about 45.3% by weight of the capsule, (B) povidone, wherein the povidone is about 5.35% by weight

of the capsule; (C) hypromellose, wherein the hypromellose is about 0.97% by weight of the capsule; and (D) sodium stearyl fumarate, wherein the sodium stearyl fumarate is about 0.34% by weight of the capsule; and

(b) one or more of a second pellet comprising:

i. a second core comprising a sugar sphere, wherein the second core is about 11.7% by weight of the capsule;
ii. A second coating comprising (A) an SGLT2 inhibitor, wherein the SGLT2 inhibitor is dapagliflozin propanediol, and wherein the dapagliflozin propanediol is about 3.7% by weight of the capsule and about 13.2% by weight of the second pellet; (B) hydroxypropyl cellulose, wherein the hydroxypropyl cellulose is about 0.48% by weight of the capsule; (C) talc, wherein the talc is about 8.2% by weight of the capsule; and (D) sodium stearyl fumarate, wherein the sodium stearyl fumarate is about 0.11% by weight of the capsule. In some embodiments, the composition is comprised in an oral dosage form. In some embodiments, the oral dosage form is a capsule.

In some embodiments, the MR modulator of the pharmaceutical composition described herein is AZD9977. In some embodiments, the pharmaceutical composition comprises about 10 mg to about 200 mg AZD9977. In some embodiments, the pharmaceutical composition comprises about 15 mg to about 100 mg AZD9977. In some embodiments, the pharmaceutical composition comprises about 15 mg to about 40 mg AZD9977. In some embodiments, the pharmaceutical composition comprises about 50 mg to about 180 mg AZD9977. In some embodiments, the pharmaceutical composition comprises about 50 mg to about 150 mg AZD9977. In some embodiments, the pharmaceutical composition comprises about 50 mg AZD9977. In some embodiments, the pharmaceutical composition comprises about 150 mg AZD9977.

In some embodiments, the SGLT2 inhibitor of the pharmaceutical composition described herein is dapagliflozin propanediol. In some embodiments, the pharmaceutical composition comprises about 5 mg to about 20 mg dapagliflozin propanediol. In some embodiments, the pharmaceutical composition comprises about 8 mg to about 18 mg dapagliflozin propanediol. In some embodiments, the pharmaceutical composition comprises about 10 mg to about 15 mg dapagliflozin propanediol. In some embodiments, the pharmaceutical composition comprises about 10 mg dapagliflozin propanediol.

In some embodiments, the pharmaceutical composition described herein comprises about 5 mg to about 20 mg active equivalent of dapagliflozin. As used herein, an "active equivalent" refers to the equivalent amount of active ingredient, dapagliflozin, that is provided by a compound, e.g., a salt or solvate of the active ingredient. As used herein, dapagliflozin propanediol provides about 81.29% w/w active equivalent of dapagliflozin. In some embodiments, 12 mg of dapagliflozin propanediol provides an active equivalent of 10 mg of dapagliflozin.

In some embodiments, the pharmaceutical composition comprises about 7 mg to about 17 mg active equivalent of dapagliflozin. In some embodiments, the pharmaceutical composition comprises about 8 mg to about 12 mg active equivalent of dapagliflozin. In some embodiments, the pharmaceutical composition comprises about 10 mg active equivalent of dapagliflozin.

In some embodiments, the pharmaceutical composition described herein comprises about 50 mg to about 200 mg AZD9977; and about 5 mg to about 20 mg dapagliflozin propanediol. In some embodiments, the pharmaceutical composition comprises about 50 mg to about 180 mg AZD9977; and about 8 mg to about 18 mg dapagliflozin propanediol. In some embodiments, the pharmaceutical composition comprises about 50 mg to about 150 mg AZD9977; and about 10 mg to about 15 mg dapagliflozin propanediol. In some embodiments, the pharmaceutical composition comprises about 50 mg AZD9977; and about 10 mg dapagliflozin propanediol. In some embodiments, the pharmaceutical composition comprises about 150 mg AZD9977; and about 10 mg dapagliflozin propanediol. In some embodiments, the composition is comprised in an oral dosage form. In some embodiments, the oral dosage form is a capsule.

In some embodiments, the pharmaceutical composition described herein comprises about 10 mg to about 50 mg AZD9977; and about 5 mg to about 20 mg dapagliflozin propanediol. In some embodiments, the pharmaceutical composition comprises about 15 mg to about 45 mg AZD9977; and about 8 mg to about 18 mg dapagliflozin propanediol. In some embodiments, the pharmaceutical composition comprises about 15 mg to about 50 mg AZD9977; and about 10 mg to about 15 mg dapagliflozin propanediol. In some embodiments, the pharmaceutical composition comprises about 15 mg AZD9977; and about 10 mg dapagliflozin propanediol. In some embodiments, the pharmaceutical composition comprises about 20 mg AZD9977; and about 10 mg dapagliflozin propanediol. In some embodiments, the pharmaceutical composition comprises about 25 mg AZD9977; and about 10 mg dapagliflozin propanediol. In some embodiments, the pharmaceutical composition comprises about 30 mg AZD9977; and about 10 mg dapagliflozin propanediol. In some embodiments, the pharmaceutical composition comprises about 35 mg AZD9977; and about 10 mg dapagliflozin propanediol. In some embodiments, the pharmaceutical composition comprises about 40 mg AZD9977; and about 10 mg dapagliflozin propanediol. In some embodiments, the pharmaceutical composition comprises about 45 mg AZD9977; and about 10 mg dapagliflozin propanediol. In some embodiments, the pharmaceutical composition comprises about 50 mg AZD9977; and about 10 mg dapagliflozin propanediol. In some embodiments, the composition is comprised in an oral dosage form. In some embodiments, the oral dosage form is a capsule.

## RELEASE PROFILE

In some embodiments, the pharmaceutical composition described herein advantageously provides comparable release profiles for both the MR modulator, e.g., AZD9977, and SGLT2 inhibitor, e.g., dapagliflozin or solvate thereof. As described herein, AZD9977 is a BCS class 4 drug due to its low solubility and low permeability, while dapagliflozin is a BCS class 3 drug, i.e., having high solubility, rapid dissolution, and low permeability. It was unexpectedly discovered that, by including the two active ingredients in separate pellets that comprise distinct excipients (e.g., core, binders, lubricants, and/or anti-tacking agents as described herein), the two active ingredients may be included in a single dosage form, such as a capsule or tablet as described herein, with comparable release profiles for both of the active ingredients. The advantages of combining two active ingredients in a single dosage form are further described herein.

In some embodiments, the pharmaceutical composition or the oral dosage form described herein releases substantially the same amount of the MR modulator and the SGLT2 inhibitor. In some embodiments, the MR modulator is AZD9977. In some embodiments, the SGLT2 inhibitor is dapagliflozin propanediol.

In some embodiments, at least 75-85% of the MR modulator is released from the pharmaceutical composition or the oral dosage form described herein within a time range of about 15-30 minutes. In other embodiments, at least 75-85% of the SGLT2 inhibitor is released from the pharmaceutical composition or the oral dosage form described herein within a time range of about 15-30 minutes. Dissolution profiles may be tested with dissolution techniques known to those skilled in the art, for example, methods such as USP 1 at 100 rpm or USP 2 at 70 rpm.

In some embodiments, the pharmaceutical composition or the oral dosage form described herein releases at least 80% of the MR modulator contained therein within 60 minutes. When tested with USP 2 at 100 rpm. In some embodiments, the pharmaceutical composition or the oral dosage form described herein releases at least 80% of the MR modulator contained therein within 30 minutes when tested with USP 2 at 100 rpm. In some embodiments, the MR modulator is AZD9977.

In some embodiments, the pharmaceutical composition or the oral dosage form described herein releases at least 80% of the SGLT2 inhibitor contained therein within 60 minutes when tested with USP 1 at 100 rpm. In some embodiments, the pharmaceutical composition or the oral dosage form described herein releases at least 80% of the SGLT2 inhibitor contained therein within 30 minutes when tested with USP 1 at 100 rpm. In some embodiments, the SGLT2 inhibitor is dapagliflozin propanediol.

## METHODS

**In** some embodiments, the present disclosure provides a method of treating heart failure in a subject in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

In some embodiments, the present disclosure provides a fixed-dose combination of an MR modulator and an SGLT2 inhibitor for use in the treatment of heart failure in a subject in need of such treatment, wherein the use comprises administering a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

**In** some embodiments, the present disclosure provides a method of treating chronic kidney disease in a subject in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

In some embodiments, the present disclosure provides a fixed-dose combination of an MR modulator and an SGLT2 inhibitor for use in the treatment of chronic kidney disease in a subject in need of such treatment, wherein the use comprises administering a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

**In** some embodiments, the present disclosure provides a method of treating heart failure and/or chronic kidney disease in a subject in need thereof, comprising administering a pharmaceutical composition or oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject in need of such treatment,

wherein the use comprises administering a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol.

In some embodiments, the present disclosure provides a method of treating heart failure and/or chronic kidney disease in a subject in need thereof, comprising administering a pharmaceutical composition or an oral dosage form described herein to the subject, wherein the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject in need of such treatment, wherein the use comprises administering a pharmaceutical composition comprising 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject with hyperkalemia (i.e., having a serum potassium level of greater than or equal to 5.5 mmol/L) in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject with hyperkalemia (i.e., having a serum potassium level of greater than or equal to 5.5 mmol/L) in need thereof, wherein the use comprises administering a pharmaceutical composition comprising an MR modulator and an SGLT2 inhibitor as described herein to the subject. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject at risk of hyperkalemia in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol. As used herein, a subject at risk of hyperkalemia includes a subject having impaired kidney function, or a subject currently prescribed at least one RAASi, or a subject having T2D, or a subject having had at least one previous hyperkalemia episodes, or a subject having a history of adverse reactions to MRAs. RAASi medicines may include angiotensin-receptor blockers, angiotensin-converting enzyme inhibitors and direct renin inhibitors that are well known in the art. In some embodiments, the subject maintains a serum potassium level of less than 5.5 mmol/L after administration of a pharmaceutical composition comprising 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject at risk of hyperkalemia in need thereof, wherein the use comprises administering a pharmaceutical composition comprising an MR modulator and an SGLT2 inhibitor as described herein to the subject. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject with recent worsening of heart failure (i.e., either stabilized in a hospital setting or the subject has been hospitalized or received acute HF treatment in an emergency facility due to heart failure within the previous 6 months) in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject with recent worsening of heart failure in need thereof, wherein the use comprises administering a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or impaired kidney function in a subject (i.e., the subject has an eGFR ranging from greater than or equal to 20 ml/min to less than or equal to 60 ml/min/1.73m$^2$) in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or impaired kidney function in a subject in need thereof, wherein the use comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating of heart failure and/or chronic kidney disease in a subject that is mineralocorticoid receptor antagonist (MRA) intolerant or considered unsuitable for MRA therapy by a physician, in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol. Common MRAs are known in the art and include, by way of non-limiting example - spironolactone, eplerenone, canrenone, progesterone, drospirenone, gestodene, and benidipine.

In some embodiments, the present disclosure provides a fixed-dose combination of an MR modulator and an SGLT2 inhibitor for use in the treatment of heart failure and/or chronic kidney disease in a subject that is mineralocorticoid receptor antagonist (MRA) intolerant or considered unsuitable for MRA therapy by a physician, in need thereof, wherein the use comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of reducing the risk of cardiovascular (CV) death and/or heart failure (HF) events in a subject in need thereof, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol. As used herein, HF events comprises hospitalization for HF and/or worsening HF events without hospitalization (see e.g., Abraham, WT et al., JACC: Heart Failure, 8:961-972 (2020), e.g., an unscheduled out-patient medical contact associated with changes in HF therapy such as:

a. The patient exhibited documented new or worsening symptoms due to HF on presentation,

b. The patient had objective evidence, i.e., physical examination findings and/or laboratory findings of new or worsening HF,

c. The patient received at least one treatment specifically for HF, and

d. The patient had improvement in symptoms and objective signs of HF in response to therapy.

In at least one embodiment, the patient experiences one or more symptoms of HF selected from the group consisting of dyspnea, decreased exercise tolerance, fatigue, and/or other symptoms of worsened end-organ perfusion or volume overload. In some embodiments where a HF event without hospitalization is required, the patient receives initiation or intensification of treatment specifically for HF. In some embodiments where a HF event without hospitalization, the patient requires significant augmentation in oral diuretic therapy, initiation of intravenous diuretic or vasoactive therapy, or mechanical fluid removal.

In some embodiments, the present disclosure provides a fixed-dose combination of an MR modulator and an SGLT2 inhibitor for use in reducing the risk of cardiovascular (CV) death and/or heart failure (HF) events in a subject in need thereof, wherein the use comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject in need thereof, wherein the method does not result in an associated risk of hyperkalemia, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject in need thereof, wherein the use does not result in an associated risk of hyperkalemia, wherein the use comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject in need thereof, wherein the method further reduces UACR in the subject, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol. In some embodiments, the reduction in UACR may be greater than 30 reduction as compared to baseline UACR, or for example, up to 25%-30% reduction as compared to baseline UACR, such as up to 30% reduction as compared to baseline UACR, for instance up to 25% reduction as compared to baseline UACR, for example up to 20% reduction as compared to baseline UACR, such as up to 15% reduction as compared to baseline UACR, for instance up to 10% reduction as compared to baseline UACR. In other embodiments, UACR reduction may be assessed by a subject transitioning from a higher to a lower UACR category, such as a subject moving from A3 category (UACR >300 mg/g) to A2 category (UACR 30-299 mg/g) or to A1 category (UACR <30 mg/g).

In some embodiments, the present disclosure provides a fixed-dose combination of an MR modulator and an SGLT2 inhibitor for use in the treatment of heart failure and/or chronic kidney disease in a subject in need thereof, wherein the use further reduces UACR in the subject, and wherein the treatment comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject in need thereof, wherein the method does not result in an associated risk of hypotension, comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a fixed-dose combination of an MR modulator and an SGLT2 inhibitor for use in the treatment of heart failure and/or chronic kidney disease in a subject in need thereof, wherein the use does not result in an associated risk of hypotension, and wherein the treatment comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In some embodiments, the present disclosure provides a method of treating heart failure and/or chronic kidney disease in a subject in need thereof, wherein the method does not result in an associated risk of acute kidney injury,

comprising administering a pharmaceutical composition or an oral dosage form described herein to the subject. In some embodiments, the composition comprises an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol. As used herein, acute kidney injury may be defined as either an investigator reported increase in serum creatinine greater than or equal to 2 times the baseline known or presumed to have occurred in the last 7 days, and/or requiring the use of temporary kidney replacement therapy for up to a period of 28 days.

In some embodiments, the present disclosure provides a fixed-dose combination of an MR modulator and an SGLT2 inhibitor for use in the treatment of heart failure and/or chronic kidney disease in a subject in need thereof, wherein the use does not result in an associated risk of acute kidney injury and wherein the treatment comprises administering to the subject a pharmaceutical composition comprising an MR modulator and an SGLT2 inhibitor as described herein. In some embodiments, the MR modulator is AZD9977. In some embodiments, the SGLT2 inhibitor is dapagliflozin or a solvate thereof, e.g., dapagliflozin propanediol. In some embodiments, the pharmaceutical composition comprises 10 mg to 50 mg of AZD9977 and 10 mg dapagliflozin or a solvate thereof, e.g., dapagliflozin propanediol.

In any of the above embodiments, the subject may have a New York Heart Association (NYHA) heart failure classification of II to IV. In at least one embodiment, the subject has a NYHA heart failure classification of II. In at least one embodiment, the subject has a NYHA heart failure classification of III or IV.

In any of the above embodiments, the subject with heart failure may include a subject having a left ventricular ejection fraction (LVEF) of greater than 50% (i.e., a subject having heart failure with preserved ejection fraction (HFpEF), or a subject having an LVEF ranging from greater or equal to 40% to less than or equal to 50% (i.e., a subject having heart failure with mid-range ejection fraction (HFmrEF), or a subject having an LVEF less than 40% (i.e., a subject having heart failure with reduced ejection fraction (HFrEF).

In any of the above embodiments, the subject with chronic kidney disease may include a subject showing kidney damage (e.g., proteinuria that may be measured by UACR, for example, a subject falling in the UACR categories A2-A3) and/or an estimated eGFR < 60 mL/min/1.73m2, persisting for at least three months.

In some embodiments, a daily dose of about 10 mg to about 200 mg, or about 15 mg to about 40 mg, or about 20 mg to about 180 mg, or about 50 mg to about 160 mg, or about 150 mg of the MR modulator is administered. In some embodiments, a daily dose of about 5 mg to about 20 mg, or about 7 mg to about 17 mg, or about 8 mg to about 12 mg, or about 10 mg of the SGLT2 inhibitor is administered. In some embodiments, a daily dose of about 10 mg to about 200 mg of the MR modulator and a daily dose of about 5 mg to about 20 mg of the SGLT2 inhibitor are administered. In some embodiments, a daily dose of about 50 mg to about 180 mg of the MR modulator and a daily dose of about 7 mg to about 17 mg of the SGLT2 inhibitor are administered. In some embodiments, a daily dose of about 50 mg to about 150 mg of the MR modulator and a daily dose of about 8 mg to about 12 mg of the SGLT2 inhibitor are administered. In some embodiments, a daily dose of about 15 mg of the MR modulator and a daily dose of about 10 mg of the SGLT2 inhibitor are administered. In some embodiments, a daily dose of about 20 mg of the MR modulator and a daily dose of about 10 mg of the SGLT2 inhibitor are administered. In some embodiments, a daily dose of about 25 mg of the MR modulator and a daily dose of about 10 mg of the SGLT2 inhibitor are administered. In some embodiments, a daily dose of about 30 mg of the MR modulator and a daily dose of about 10 mg of the SGLT2 inhibitor are administered. In some embodiments, a daily dose of about 35 mg of the MR modulator and a daily dose of about 10 mg of the SGLT2 inhibitor are administered. In some embodiments, a daily dose of about 40 mg of the MR modulator and a daily dose of about 10 mg of the SGLT2 inhibitor are administered. In some embodiments, a daily dose of about 50 mg of the MR modulator and a daily dose of about 10 mg of the SGLT2 inhibitor are administered. In some embodiments, a daily dose of about 150 mg of the MR modulator and a daily dose of about 10 mg of the SGLT2 inhibitor are administered. In some embodiments, the MR modulator is AZD9977. In some embodiments, the SGLT2 inhibitor is dapagliflozin.

In some embodiments, the pharmaceutical composition comprises: about 10 mg to about 200 mg AZD9977; and about 5 mg to about 20 mg active equivalent of dapagliflozin. In some embodiments, the pharmaceutical composition comprises about 20 mg to about 180 mg AZD9977; and about 7 mg to about 17 mg active equivalent of dapagliflozin. In some embodiments, the pharmaceutical composition comprises about 50 mg to about 160 mg AZD9977; and about 8 mg to about 12 mg active equivalent of dapagliflozin. In some embodiments, the pharmaceutical composition comprises about 150 mg AZD9977; and about 10 mg active equivalent of dapagliflozin. In some embodiments, the pharmaceutical composition comprises about 50 mg AZD9977; and about 10 mg active equivalent of dapagliflozin. In some embodiments, a daily dose of about 15 mg of AZD9977 and a daily dose of about 10 mg of dapagliflozin are administered. In some embodiments, a daily dose of about 20 mg of AZD9977and a daily dose of about 10 mg of dapagliflozin are administered. In some embodiments, a daily dose of about 25 mg of AZD9977 and a daily dose of about 10 mg of dapagliflozin are administered. In some embodiments, a daily dose of about 30 mg of AZD9977and a daily dose of about 10 mg of dapagliflozin are administered. In some embodiments, a daily dose of about 35 mg of AZD9977and a daily dose of about 10 mg of dapagliflozin are administered. In some embodiments, a daily dose of about 40 mg of AZD9977 and a daily dose of

about 10 mg of dapagliflozin are administered. In some embodiments, dapagliflozin is in the form of dapagliflozin propanediol. the composition is comprised in an oral dosage form. In some embodiments, the oral dosage form is a capsule.

In some embodiments, the method comprises administering a pharmaceutical composition comprising:

(a) one or more of a first pellet, comprising:

i. a first core comprising a microcrystalline cellulose core, wherein the first core is about 10% to about 30%, optionally about 10% to about 25%, by weight of the composition;

ii. a first coating comprising

(A) about 20% to about 50% by weight of the composition of an MR modulator, wherein the MR modulator is AZD9977;

(B) about 1% to about 10%, optionally about 4% to about 8%, by weight of the composition of povidone; and

(C) about 0.01% to about 0.5%, optionally about 0.2% to about 0.4%, by weight of the composition of sodium stearyl fumarate; and

(b) one or more of a second pellet comprising:

i. a second core comprising a sugar sphere, wherein the sugar sphere is about 10% to about 40%, optionally about 15% to about 35%, by weight of the composition;

ii. a second coating comprising

(A) about 1% to about 10%, optionally about 3% to about 7%, by weight of the composition of an SGLT2 inhibitor, wherein the SGLT2 inhibitor is dapagliflozin, and wherein the SGLT2 is about 5% to about 20%, optionally about 5% to about 15%, by weight of the second pellet;

(B) about 0.1% to about 5%, optionally about 0.4% to about 1%, by weight of the composition of hydroxypropyl cellulose;

(C) about 1% to about 20%, optionally about 7% to about 16%, by weight of the composition of talc; and

(D) about 0.01% to about 1%, optionally about 0.05% to about 0.2%, by weight of the composition of sodium stearyl fumarate.

In some embodiments, the composition is comprised in an oral dosage form. In some embodiments, the oral dosage form is a capsule.

In some embodiments, the method of treating heart failure and/or chronic kidney disease comprises administering a pharmaceutical composition comprising:

(a) one or more of a first pellet, comprising:

i. a first core comprising a microcrystalline cellulose core, wherein the first core is about 20.7% by weight of the composition;

ii. a first coating comprising

(A) about 40% by weight of the composition of an MR modulator, wherein the MR modulator is AZD9977;

(B) about 6%, by weight of the composition of povidone; and

(C) about 0.1% by weight of the composition of sodium stearyl fumarate; and

(b) one or more of a second pellet comprising:

i. a second core comprising a sugar sphere, wherein the sugar sphere is about 21.9% by weight of the composition;

ii. a second coating comprising

(A) about 3.3% by weight of the composition of an SGLT2 inhibitor, wherein the SGLT2 inhibitor is dapagliflozin propanediol, and wherein the SGLT2 is about 12.8% by weight of the second pellet;

(B) about 0.43% by weight of the composition of hydroxypropyl cellulose;

(C) about 7.2% by weight of the composition of talc; and

(D) about 0.15% by weight of the composition of sodium stearyl fumarate.

In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet, comprising:

i. a first core comprising a microcrystalline cellulose core, wherein the first core is about 5% to about 25% by weight of the capsule;
ii. a first coating comprising (A) an MR modulator, wherein the MR modulator is AZD9977, and wherein the AZD9977 is about 10% to about 45% by weight of the capsule; (B) povidone, wherein the povidone is about 1% to about 10% by weight of the capsule; and (C) sodium stearyl fumarate, wherein the sodium stearyl fumarate is about 0.01% to about 1% by weight of the capsule; and

(b) one or more of a second pellet comprising:

i. a second core comprising a sugar sphere, wherein the sugar sphere is about 5% to about 30% by weight of the capsule;
ii. a second coating comprising (A) an SGLT2 inhibitor, wherein the SGLT2 inhibitor is dapagliflozin propanediol, and wherein the dapagliflozin propanediol is about 1% to about 10% by weight of the capsule; (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 0.1% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet, comprising:

i. a first core comprising a microcrystalline cellulose core, wherein the first core is about 5% to about 20% by weight of the capsule;
ii. a first coating comprising (A) an MR modulator, wherein the MR modulator is AZD9977, and wherein the AZD9977 is about 15% to about 40% by weight of the capsule; (B) povidone, wherein the povidone is about 2% to about 6% by weight of the capsule; and (C) sodium stearyl fumarate, wherein the sodium stearyl fumarate is about 0.02% to about 0.4% by weight of the capsule; and

(b) one or more of a second pellet comprising:

i. a second core comprising a sugar sphere, wherein the sugar sphere is about 10% to about 22% by weight of the capsule;
ii. a second coating comprising (A) an SGLT2 inhibitor, wherein the SGLT2 inhibitor is dapagliflozin, and wherein the dapagliflozin is about 2% to about 5% by weight of the capsule; (B) hydroxypropyl cellulose, wherein the hydroxypropyl cellulose is about 0.4% to about 0.6% by weight of the capsule; (C) talc, wherein the talc is about 5% to about 12% by weight of the capsule; and (D) sodium stearyl fumarate, wherein the sodium stearyl fumarate is about 0.02% to about 0.08% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet, comprising:

i. a first core comprising a microcrystalline cellulose core, wherein the first core is about 18% by weight of the capsule;
ii. a first coating comprising (A) an MR modulator, wherein the MR modulator is AZD9977, and wherein the AZD9977 is about 35% by weight of the capsule; (B) povidone, wherein the povidone is about 5.3% by weight of the capsule; and (C) sodium stearyl fumarate, wherein the sodium stearyl fumarate is about 0.12% by weight of

the capsule; and

(b) one or more of a second pellet comprising:

i. a second core comprising a sugar sphere, wherein the sugar sphere is about 12.8% by weight of the capsule;
ii. a second coating comprising (A) an SGLT2 inhibitor, wherein the SGLT2 inhibitor is dapagliflozin, and wherein the dapagliflozin is about 2.9% by weight of the capsule; (B) hydroxypropyl cellulose, wherein the hydroxypropyl cellulose is about 0.38% by weight of the capsule; (C) talc, wherein the talc is about 6.3% by weight of the capsule; and (D) sodium stearyl fumarate, wherein the sodium stearyl fumarate is about 0.04% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet, comprising:

i. a first core comprising a microcrystalline cellulose core, wherein the first core is about 9.9% by weight of the capsule;
ii. a first coating comprising (A) an MR modulator, wherein the MR modulator is AZD9977, and wherein the AZD9977 is about 19.2% by weight of the capsule; (B) povidone, wherein the povidone is about 2.9% by weight of the capsule; and (C) sodium stearyl fumarate, wherein the sodium stearyl fumarate is about 0.06% by weight of the capsule; and

(b) one or more of a second pellet comprising:

i. a second core comprising a sugar sphere, wherein the sugar sphere is about 21% by weight of the capsule;
ii. a second coating comprising (A) an SGLT2 inhibitor, wherein the SGLT2 inhibitor is dapagliflozin, and wherein the dapagliflozin is about 4.7% by weight of the capsule; (B) hydroxypropyl cellulose, wherein the hydroxypropyl cellulose is about 0.63% by weight of the capsule; (C) talc, wherein the talc is about 10.4% by weight of the capsule; and (D) sodium stearyl fumarate, wherein the sodium stearyl fumarate is about 0.07% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet comprising:

i. a first core comprising a microcrystalline cellulose core, wherein the first core is about 5% to about 25% by weight of the capsule;
ii. a first coating comprising (A) an MR modulator, wherein the MR modulator is AZD9977, and wherein the AZD9977 is about 10% to about 45% by weight of the capsule, (B) povidone, wherein the povidone is about 2% to about 8% by weight of the capsule; (C) hypromellose, wherein the hypromellose is about 0.5% to about 3% by weight of the capsule; and (D) sodium stearyl fumarate, wherein the sodium stearyl fumarate is about 0.01% to about 0.5% by weight of the capsule; and

(b) one or more of a second pellet comprising:

i. a second core comprising a sugar sphere, wherein the second core is about 5% to about 30% by weight of the capsule;
ii. a second coating comprising (A) an SGLT2 inhibitor, wherein the SGLT2 inhibitor is dapagliflozin propanediol, and wherein the dapagliflozin propanediol is about 1% to about 10% by weight of the capsule and about 5% to about 20% by weight of the second pellet, (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 1% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet comprising:

i. a first core comprising a microcrystalline cellulose core, wherein the first core is about 23.9% by weight of the capsule;
ii. a first coating comprising (A) an MR modulator, wherein the MR modulator is AZD9977, and wherein the AZD9977 is about 45.3% by weight of the capsule, (B) povidone, wherein the povidone is about 5.35% by weight of the capsule; (C) hypromellose, wherein the hypromellose is about 0.97% by weight of the capsule; and (D) sodium stearyl fumarate, wherein the sodium stearyl fumarate is about 0.34% by weight of the capsule; and

(b) one or more of a second pellet comprising:

i. a second core comprising a sugar sphere, wherein the second core is about 11.7% by weight of the capsule;
ii. a second coating comprising (A) an SGLT2 inhibitor, wherein the SGLT2 inhibitor is dapagliflozin propanediol, and wherein the dapagliflozin propanediol is about 3.7% by weight of the capsule and about 13.2% by weight of the second pellet; (B) hydroxypropyl cellulose, wherein the hydroxypropyl cellulose is about 0.48% by weight of the capsule; (C) talc, wherein the talc is about 8.2% by weight of the capsule; and (D) sodium stearyl fumarate, wherein the sodium stearyl fumarate is about 0.11% by weight of the capsule. In some embodiments, the composition is comprised in an oral dosage form. In some embodiments, the oral dosage form is a capsule.

In some embodiments, the method of treating heart failure and/or chronic kidney disease comprises administering a pharmaceutical composition in the form of a capsule, comprising:

(a) one or more of a first pellet comprising:

i. a first core comprising a microcrystalline cellulose core, wherein the first core is about 56.2 % by weight of the capsule;
ii. a first coating comprising (A) an MR modulator, wherein the MR modulator is AZD9977, and wherein the AZD9977 is about 11.2% by weight of the capsule, (B) hypromellose, wherein the hypromellose is about 0.22% by weight of the capsule; and (C) sodium stearyl fumarate, wherein the sodium stearyl fumarate is about 0.37% by weight of the capsule; and

(b) one or more of a second pellet comprising:

i. a second core comprising a sugar sphere, wherein the second core is about 26.3% by weight of the capsule;
ii. a second coating comprising (A) an SGLT2 inhibitor, wherein the SGLT2 inhibitor is dapagliflozin propanediol, and wherein the dapagliflozin propanediol is about 2.8% by weight of the capsule and about 10.7% by weight of the second pellet; (B) hydroxypropyl cellulose, wherein the hydroxypropyl cellulose is about 0.37% by weight of the capsule; (C) talc, wherein the talc is about 6.2% by weight of the capsule; and (D) sodium stearyl fumarate, wherein the sodium stearyl fumarate is about 0.34% by weight of the capsule. In some embodiments, the composition is comprised in an oral dosage form. In some embodiments, the oral dosage form is a capsule.

In some embodiments, the method comprises once daily administrating of the pharmaceutical composition or oral dosage form described herein. In some embodiments, the method comprises 2x daily, 3x daily, 4x daily, 5x daily, 6x daily, or more frequent administration of the pharmaceutical composition or oral dosage form described herein. One of ordinary skill in the art would understand that, if the pharmaceutical composition is administered more than once daily, the amounts of the MR modulator (e.g., AZD9977) and SGLT2 inhibitor (e.g., dapagliflozin) in the pharmaceutical composition shall be adjusted accordingly to maintain the desired daily dose. For example, to achieve a daily dose of about 50 mg or about 150 mg of the MR modulator and about 10 mg of the SGLT2 inhibitor, a composition or oral dosage form for twice daily administration should include 22.5 mg or 75 mg MR modulator and 5 mg SGLT2 inhibitor.

In some embodiments, the pharmaceutical composition or the oral dosage form is administered to the subject in need thereof in a fasted state. As defined by the US Food and Drug Administration, a treatment in a "fasted state" occurs when there is a fast lasting at least 10 hours, e.g., an overnight fasting and with no food consumed until after at least 4 hours after drug dosing. In contrast, a treatment in a "fed state" occurs starting with the same overnight 10 hour fast, but the subject consumes a meal within 30 minutes before drug dosing, with no additional food consumption for at least 4 hours after drug dosing. See, e.g., U.S. Food and Drug Administration, Center for Drug Evaluation and Research (CDER),

"Guidance for Industry - Food-Effect Bioavailability and Fed Bioequivalence Studies," December 2002.

In some embodiments, the pharmaceutical composition or oral dosage form comprising the MR modulator (e.g., AZD9977) and the SGLT2 inhibitor (e.g., dapagliflozin or solvate thereof), when administered in the fasted state, provides bioequivalence to the MR modulator and the SGLT2 inhibitor administered in separate dosage forms. Methods of evaluating bioequivalence are known to one of ordinary skill in the art. See, e.g., U.S. Food and Drug Administration, Center for Drug Evaluation and Research (CDER), "Guidance for Industry - Bioavailability and Bioequivalence Studies Submitted in NDAs or INDs-General Considerations - Draft Guidance," March 2014.

In some embodiments, the pharmaceutical composition or oral dosage form described herein is bioequivalent to separate dosage forms of the MR modulator and SGLT2 inhibitor as measured by one or both of $AUC_{last}$ and $AUC_{inf}$. As used herein, "$AUC_{last}$" refers to the area under the curve of the drug concentration from the time of dosing to the time of the last measurable (positive) concentration ($T_{last}$). As used herein, "$AUC_{inf}$" refers to the area under the curve of the drug concentration from the time of dosing extrapolated to infinity and is a theoretical measurement of the total exposure of the drug to the body from administration until complete elimination.

In some embodiments, an $AUC_{last}$ and $AUC_{inf}$ of the subject following administration of the pharmaceutical composition or oral dosage form as described herein are within about 1% to about 20% of an $AUC_{last}$ and $AUC_{inf}$ of the subject administered with separate dosage forms of the MR modulator and the SGLT2 inhibitor. In some embodiments, an $AUC_{last}$ and $AUC_{inf}$ of the subject following administration of the pharmaceutical composition or oral dosage form as described herein are within about 5% to about 15% of an $AUC_{last}$ and $AUC_{inf}$ of the subject administered with separate dosage forms of the MR modulator and the SGLT2 inhibitor. In some embodiments, an $AUC_{last}$ and $AUC_{inf}$ of the subject following administration of the pharmaceutical composition or oral dosage form as described herein are within 10% of an $AUC_{last}$ and $AUC_{inf}$ of the subject administered with separate dosage forms of the MR modulator and the SGLT2 inhibitor. In some embodiments, an $AUC_{last}$ and $AUC_{inf}$ of the subject following administration of the pharmaceutical composition or oral dosage form as described herein are within 10%, within 8%, within 5%, or within 2% of an $AUC_{last}$ and $AUC_{inf}$ of the subject administered with separate dosage forms of the MR modulator and the SGLT2 inhibitor. In some embodiments, an $AUC_{last}$ and $AUC_{inf}$ of the subject following administration of the pharmaceutical composition or oral dosage form as described herein are substantially the same as an $AUC_{last}$ and $AUC_{inf}$ of the subject administered with separate dosage forms of the MR modulator and the SGLT2 inhibitor.

In some embodiments, the subject in need thereof is diagnosed with chronic heart failure (HF), chronic kidney disease (CKD), or both. In some embodiments, the subject in need thereof is diagnosed with chronic HF and is at elevated risk for developing CKD; or diagnosed with CKD and is at elevated risk for developing chronic HF. In some embodiments, the subject has a family history of chronic HF, CKD, or both. In some embodiments, the subject is a healthy subject enrolled in a clinical trial for the pharmaceutical composition or oral dosage form described herein.

In some embodiments, the present disclosure provides a method of making a capsule comprising the pharmaceutical composition described herein, comprising:

(1) forming the first pellet, comprising:

a) combining a first binder with water to form a first binder/water solution;
b) adding an MR modulator to the first binder/water solution to form a first suspension;
c) optionally, sieving the first suspension through a 200-300 μm sieve;
d) coating a first core with the sieved first suspension to form a first coated core;
e) mixing the first coated core with a first lubricant to form a first pellet;

(2) forming a second pellet, comprising:

a) combining a second binder with water to form a second binder/water solution;
b) adding an SGLT2 inhibitor and an anti-tacking agent to the second binder/water solution to form a second suspension;
c) coating a second core with the second suspension to form a second coated core;
d) mixing the second coated core with a second lubricant to form a second pellet;

(3) combining the first pellet and the second pellet in a capsule.

Components of the method are further described herein. In some embodiments, the first binder comprises povidone or povidone and hypromellose; the MR modulator comprises AZD9977; the first core comprises microcrystalline cellulose; and/or the first lubricant comprises sodium stearyl fumarate. In some embodiments, the second binder comprises hydroxypropyl cellulose; the SGLT2 inhibitor comprises dapagliflozin propanediol; the anti-tacking agent comprises talc; the second core comprises a sugar sphere; and/or the second lubricant comprises sodium stearyl

fumarate. In some embodiments, the first pellet comprises at least two first binders, wherein steps (1)(a) to (1)(d) are repeated for each first binder, thereby coating the first core with at least two layers comprising each first binder and the MR modulator. In some embodiments, the coating comprises spray coating. Spray coating processes are known to one of ordinary skill in the art.

In some embodiments, the present disclosure provides a method of making a capsule comprising the pharmaceutical composition described herein, comprising:

(1) forming the first pellet, comprising:

a) combining povidone with water to form a povidone/water solution;
b) adding AZD9977 to the povidone/water solution to form a first suspension, wherein the first suspension comprises about 20% to about 30% w/w AZD9977;
c) sieving or wet milling the first suspension through a 200-300 μm sieve;
d) coating a microcrystalline cellulose core with the sieved first suspension to form a first coated core;
e) mixing the first coated core with sodium stearyl fumarate to form a first pellet;

(2) forming a second pellet, comprising:

a) combining hydroxypropyl cellulose with water to form an HPC/water solution;
b) adding dapagliflozin propanediol and talc to the HPC/water solution to form a second suspension, wherein the second suspension comprises about 10% to about 15% dapagliflozin propanediol;
c) coating a sugar sphere core with the second suspension to form a second coated core;
d) mixing the second coated core with sodium stearyl fumarate to form a second pellet;

(3) combining the first pellet and the second pellet in a capsule.

In some embodiments, the present disclosure provides a method of making a capsule comprising the pharmaceutical composition described herein, comprising:

(1) forming the first pellet, comprising:

a) combining hypromellose with water to form a hypromellose/water solution;
b) adding AZD9977 to the hypromellose/water solution to form a suspension, wherein the suspension comprises about 20% to about 30% w/w AZD9977;
c) sieving the suspension of (b) through a 200-300 μm sieve;
d) coating a microcrystalline cellulose core with the sieved suspension of (c) to form a first coated core comprising a first layer;
e) combining povidone with water to form a povidone/water solution;
f) adding AZD9977 to the povidone/water solution to form a suspension, wherein the suspension comprises about 20% to about 30% w/w AZD9977;
g) sieving the suspension of (f) through a 200-300 μm sieve;
h) coating the first coated core comprising the first layer with the sieved suspension of (g), thereby forming a first coated core comprising first and second layers;
g) mixing the first core comprising the first and second layers with sodium stearyl fumarate to form a first pellet;

(2) forming a second pellet, comprising:

a) combining hydroxypropyl cellulose with water to form an HPC/water solution;
b) adding dapagliflozin propanediol and talc to the HPC/water solution to form a second suspension;
c) coating a sugar sphere core with the second suspension to form a second coated core;
d) mixing the second coated core with sodium stearyl fumarate to form a second pellet;

(3) combining the first pellet and the second pellet in a capsule.

In some embodiments, steps (1) and (2) of the method described herein are performed in parallel. In some embodiments, steps (1) and (2) of the method are performed sequentially and in any order.

The entire contents of all publications, patents, and patent applications referenced herein are hereby incorporated herein by reference.

The specific examples included herein are for illustrative purposes only and are not to be considered as limiting to this disclosure. Moreover, the compositions, systems and methods provided herein have been described in relation to certain embodiments thereof, and many details have been set forth for purposes of illustration. It will be apparent to those skilled in the art that the disclosure is susceptible to additional embodiments and that certain of the details described herein may be varied without departing from the basic principles of the disclosure. Any active agents and reagents used in the following examples are either commercially available or, with the benefit of the descriptions provided herein, can be prepared according to standard literature procedures by those skilled in the art.

## EXAMPLES

### EXAMPLE 1. Preparation of AZD9977 + Dapagliflozin Capsules

#### Immediate Release (IR) dapagliflozin pellets:

**Preparation of dapagliflozin spray coating suspension for low drug-load pellet (D1 Suspension).** 7.5 g of hydroxypropylcellulose (HPC) SSL (Nisso Soda) was added to 1312.8 g of purified water and left stirring for approximately 2 hours (until clear solution). 123.8 g of Talc and 56.25 g of dapagliflozin (Dottikon) was added to the HPC SSL/water solution to produce a 12.5 % (w/w) dapagliflozin coating suspension that was left stirring overnight at RT.

**Spray coating of 85 mg/g dapagliflozin IR pellets (D1).** 300 g of Non-pareil cores (VIVAPHARM®, JRS Pharma) was loaded into a Wurster fluid bed spray coater (Graniten Engineering). The cores were coated with 1207 g of the dapagliflozin coating suspension at the conditions summarized in Table 1 to produce 400 g of dapagliflozin IR pellets with 83.5 mg/g drug load.

**Preparation of dapagliflozin spray coating suspension for high drug-load pellet (D2 Suspension).** 5.0 g of HPC SSL (Nisso Soda) was added to 875.7 g of purified water and left stirring for approximately 2 hours (until clear solution). 82.5 g of Talc and 37.5 g of dapagliflozin (Dottikon) was added to the HPC SSL/water solution to produce an 12.5 % (w/w) dapagliflozin coating suspension that was left stirring overnight at RT.

**Spray coating of 130 mg/g dapagliflozin IR pellets (D2).** 100 g of Non-pareil cores (VIVAPHARM®, JRS Pharma) was loaded into a Wurster fluid bed spray coater (Graniten Engineering). The cores were coated with 847.7 g of the dapagliflozin coating suspension at the conditions summarized in Table 1 to produce 174 g of dapagliflozin IR pellets with 131.5 mg/g drug load.

**Preparation of dapagliflozin spray coating suspension for dapa-pellet (D3 Suspension).** 0.9 kg of HPC SSL (Nisso Soda) was added to 67.5 kg of purified water and left stirring for approximately 1 hour (until clear solution). 14.85 kg of Talc and 6.75 kg of dapagliflozin (Dottikon) was added to the HPC SSL/water solution to produce an 25 % (w/w) dapagliflozin coating suspension that was left stirring overnight at RT.

**Spray coating of 105 mg/g dapagliflozin IR pellets (D3).** 30 kg of Non-pareil cores (VIVAPHARM®, JRS Pharma) was loaded into a Wurster fluid bed spray coater (GPCG30, Glatt). The cores were coated with 90 kg of the dapagliflozin coating suspension at the conditions summarized in Table 1 to produce 49.5 kg of dapagliflozin IR pellets with 107 mg/g drug load.

#### Immediate Release (IR) AZD9977 pellets:

**Preparation of AZD9977 spray coating suspension (A1 suspension).** 60 g of polyvinylpyrrolidone (povidone; PVP) K30 (BASF) was added to 1540 g of purified water and left stirring for approximately 1 hour (until clear solution). 400 g of AZD9977 (STA WuXi) was added to the PVPK30/water solution to produce a 23 % (w/w) AZD9977 coating suspension that was left stirring overnight at RT. The suspension was sieved through a 250 μm before use in coating step.

**Spray coating of 600 mg/g AZD9977 IR pellets (A1).** 170 g of microcrystalline cellulose (MCC) cores (VIVAPUR® 100, JRS Pharma) was loaded into a Wurster fluid bed spray coater (Graniten Engineering). The cores were coated with 1653 g of the AZD9977 coating suspension 1 at the conditions summarized in Table 1 to produce 532 g of AZD9977 IR pellets 1 with 598 mg/g drug load.

**Preparation of AZD9977 spray coating suspension (A2a suspension - layer 1).** 12 g of hydroxypropyl methycellulose (HPMC) 6 cps (Dow Chem) was added to 468 g of purified water and left stirring for approximately 1 hour (until clear solution). 120 g of AZD9977 (STA WuXi) was added to the HPMC/water solution to produce an 22 % (w/w) AZD9977 coating suspension A2a that was left stirring overnight at RT. The suspension was sieved through a 250 μm before use in coating step.

**Spray coating of 320 mg/g AZD9977 IR pellets (A2a - first layer).** 200 g of MCC cores (VIVAPUR® 100, JRS Pharma) was loaded into a Wurster fluid bed spray coater (Graniten Engineering). The cores were coated with 504 g of the AZD9977 coating suspension A2a at the conditions summarized in Table 1 to produce 293 g of AZD9977 IR pellets A2a with 323 mg/g drug load.

**Preparation of AZD9977 spray coating suspension (A2 suspension - layer 2).** 45 g of PVP K30 (BASF) was added to 1155 g of purified water and left stirring for approximately 30 min (until clear solution). 300 g of AZD9977 (STA WuXi) was added to the PVP K30/water solution to produce an 23 % (w/w) AZD9977 suspension that was left stirring overnight at RT. The suspension was sieved through a 250 µm before use in coating step.

**Spray coating of 600 mg/g AZD9977 IR pellets (A2 - second layer).** 265 g of AZD9977 IR pellets A2a was loaded into a Wurster fluid bed spray coater (Graniten Engineering). The cores were coated with 1266 g of the AZD9977 coating suspension A2 at the conditions summarized in Table 1 to produce 547 g of AZD9977 IR pellets A2 with 605 mg/g drug load.

**Preparation of AZD9977 spray coating suspension (A3 suspension)** 1.0 kg of hydroxypropyl methylcellulose (HPMC) 6 cps (Dow Chem) was added to 39 kg of purified water and left stirring for at least 0.5 h (until clear solution). 10 kg of AZD9977 (STA WuXi) was added to the HPMC/water solution to produce a 22 % (w/w) AZD9977 coating suspension that was left stirring overnight at RT.

**Spray coating of 200 mg/g AZD9977 IR pellets (A3 - second layer)** 39 kg of microcrystalline cellulose (MCC) cores (VIVAPUR® 200, JRS Pharma) was loaded into a Wurster fluid bed spray coater (GPCG30). The cores were coated with 50 kg of the AZD9977 coating suspension at the conditions summarized in Table below to produce 49.0 kg of AZD9977 IR pellets with 202 mg/g drug load.

**Table 1. Spray Coating Process Parameters.**

| Process Parameter | Dapa Pellets (D1) | Dapa Pellets D2 | Dapa Pellets D3 | AZD997 7 Pellet A1 | AZD997 7 Pellet A2a | AZD997 7 Pellet A2 | AZD9977 Pellet A3 | Unit |
|---|---|---|---|---|---|---|---|---|
| Inlet fluidising gas temp | 87 - 83 | 85-83 | 98 | 80-77 | 72-69 | 80-77 | 73 | [°C] |
| Outlet fluidising gas temp | 69 - 48.7 | 64-49 | 63-45 | 48-39 | 51-38 | - 48-40 | 33-43 | [°C] |
| Fluidising gas flow | Appro x. 60 | App.60 | Approx 1254 | Approx. 50 | Approx. 35 | Approx. 50 | Approx. 730 | [$Nm^3$/ h] |
| Water amount in fluidising gas | 6.0 | 6.0 | 6.0 | 6.0 | 15 | 6.0 | 6.0 | [g/kg] |
| Atomizer flow | Appro x. 4.2 | App.4. 2 | Approx . 4.0 | Approx. 2.3 | Approx. 2.5 | Approx. 2.3 | Approx. 51 | [$Nm^3$/ h] |
| Atomizer pressure | Appro x. 2 | App.4. 2 | Approx . 4.0 | Approx. 1.9 | Approx. 2.2 | Approx. 1.9 | Approx. 2.2 | [bar] |
| Average spray rate | 17.1 | 17.3 | Approx . 500 | 20 | 12 | 20 | Approx. 250 | [g/min ] |

**Filling of D1, D2, D3, A1, A2, A3 Pellets into Capsules**

**Lubrication:**

**A1:** 518 g of the AZD9977 pellets A1 were mixed with 2.34 g of sodium stearyl fumarate in a Turbula T2F blender for 10 min/ 23 rpm to produce 520.6 g of lubricated AZD9977 pellets.

**A2:** 543 g of the AZD9977 pellets A2 were mixed with 2.45 g of sodium stearyl fumarate in a Turbula T2F blender for 10 min/ 23 rpm to produce 544.7 g of lubricated AZD9977 pellets.

**D1**:-388.5 g of the dapagliflozin pellets D1 were mixed with 1.76g sodium stearyl fumarate in a Turbula T2F blender for 10 min /23 rpm to produce 390.3 g lubricated dapagliflozin DL low pellets.

**D2:** 167.3 g of the dapagliflozin pellets D2 were mixed with 0.76g sodium stearyl fumarate in a Turbula T2F blender for 10 min /23 rpm to produce 168.1 g lubricated dapagliflozin DL high pellets.

D3: 167.3 g of the dapagliflozin pellets D3 were mixed with 0.76g sodium stearyl fumarate in a Turbula T2F blender for 10 min /23 rpm to produce 168.1 g lubricated dapagliflozin DL pellets.

**FDC Capsules** (also depicted in FIG. 2A):

**Capsule 1 ("FDC1"): Dose 150 mg AZD9977 pellets A1 and 10 mg Dapagliflozin D1.** 250 g of lubricated AZD9977 pellets A1 and 124 g dapagliflozin pellets D1 were filled into 1000 white, size 0 gelatin capsules (Capsugel) with an average fill weight of 250 mg AZD9977 pellets and 124 mg dapagliflozin pellets/ capsule using a JuBo capsule filler.

**Capsule 2 ("FDC2"): Dose 150 mg AZD9977 pellets A2 and 10 mg Dapagliflozin D2.** 250 g of lubricated AZD9977 pellets A2 and 80 g dapagliflozin pellets D2 were filled into 1000 white, size 0 gelatin capsules (Capsugel) with an average fill weight of 250 mg AZD9977 pellets and 80 mg dapagliflozin pellets/ capsule using a JuBo capsule filler.

**Capsule 3 ("FDC3"): Dose 150 mg AZD9977 pellets A1 and 10 mg Dapagliflozin D2.** 250 g of lubricated AZD9977 pellets A1 and 80 g dapagliflozin pellets D2 were filled into 1000 white, size 0 gelatin capsules (Capsugel) with an average fill weight of 250 mg AZD9977 pellets and 80 mg dapagliflozin pellets/ capsule using a JuBo capsule filler.

**Capsule 4 ("FDC4"): Dose 150 mg AZD9977 pellets A2 and 10 mg Dapagliflozin D1.** 250 g of lubricated AZD9977 pellets A2 and 124 g dapagliflozin pellets D1 were filled into 1000 white, size 0 gelatin capsules (Capsugel) with an average fill weight of 250 mg AZD9977 pellets and 124 mg dapagliflozin pellets/ capsule using a JuBo capsule filler.

**Capsule 5: Dose 40 mg AZD9977 pellets A3 and 10 mg Dapagliflozin D3.** 201 g of lubricated AZD9977 pellets A3 and 93.8 g dapagliflozin pellets D3 were filled into 1000 white, size 2 gelatin capsules (Capsugel) with an average fill weight of 200 mg AZD9977 pellets and 93.4 mg dapagliflozin pellets/ capsule using a JuBo capsule filler.

**EXAMPLE 2. Dissolution Studies**

The FDC formulations (Capsules 1-4) as described in Example 1 were subjected to in-vitro dissolution studies.

Results are shown in FIGS. 1A (dapagliflozin, tested in USP apparatus 1 at 100 rpm), 1B (AZD9977, tested in USP apparatus 2 at 100 rpm), and 1C (AZD9977, tested in USP apparatus 1 at 100 rpm). As shown in FIG. 1A, all four FDC formulations released >80% of the dapagliflozin within 30 minutes, as tested in USP apparatus 1 at 100 rpm. As shown in FIG. 1B, all four FDC formulations released >80% of the AZD9977 within 30 minutes, as tested in USP apparatus 2 at 100 rpm. As shown in FIG. 1C, FDC 1 and FDC 3, containing povidone-coated AZD9977 pellets, released >80% of the AZD9977 within 30 minutes, as tested in USP apparatus 1 at 100 rpm; and FDC 2 and FDC 4, containing HPMC/povidone-coated AZD9977 pellets, released >60% of the AZD9977 within 30 minutes, as tested in USP apparatus 1 at 100 rpm.

**EXAMPLE 3. Clinical Study for Evaluation of Bioavailability of AZD9977 and Dapagliflozin FDC Capsules**

A randomized, parallel group, open-label, four/five period, eight treatment, single-dose, cross-over study was conducted in healthy volunteers to evaluate the bioavailability of AZD9977 and dapagliflozin FDC formulations (Capsules 1-4) as described in Example 1. Subjects were randomized to one of the 8 treatment sequences as depicted in FIG. 2B. Treatments are as follows:

Group 1:

Treatment A1 = 150 mg AZD9977 (AZD9977 capsule 50 mg + AZD9977 capsule 100 mg) and 10 mg dapagliflozin tablet, fasted;

Treatment B = 150 mg AZD9977 + 10 mg dapagliflozin Capsule 1, fasted;

Treatment C = 150 mg AZD9977 + 10 mg dapagliflozin Capsule 1, fed;

Treatment D = 150 mg AZD9977 + 10 mg dapagliflozin Capsule 2, fasted;

Treatment E = 150 mg AZD9977 + 10 mg dapagliflozin Capsule 2, fed;

Group 2:

Treatment A2 = 150 mg AZD9977 (AZD9977 capsule 50 mg + AZD9977 capsule 100 mg) and 10 mg dapagliflozin tablet, fasted;

Treatment F = 150 mg AZD9977 + 10 mg dapagliflozin Capsule 3, fasted;

Treatment G = 150 mg AZD9977 + 10 mg dapagliflozin Capsule 4, fasted;

Treatment H = 10 mg dapagliflozin capsule, fasted.

**Plasma AZD9977 concentrations following single oral administration of different formulations of AZD9977 and dapagliflozin.**

Mean plasma AZD9977 concentration versus time curves are presented in FIG. 3A (linear scale) and FIG. 3B (semi logarithmic scale).

Low to moderate between-subject variability in AUCs was observed across all treatments with geometric mean coefficient of variation ranging from approximately 20% to 28%. Low to high between-subject variability in Cmax was observed across all treatments with geometric mean coefficient of variation ranging from approximately 15% to 50%.

The AZD9977 AUClast and AUCinf were comparable between Treatment B (150 mg AZD9977 + 10 mg dapagliflozin Capsule 1, fasted) and the reference, Treatment A1 (150 mg AZD9977 (AZD9977 capsule 50 mg + AZD9977 capsule 100 mg) and 10 mg dapagliflozin tablet, fasted). The Cmax was possibly 14% lower than the reference treatment and the C24 value was 49% higher than the reference treatment. See FIG. 4A.

The AZD9977 AUClast and AUCinf were comparable between Treatment D (150 mg AZD9977 + 10 mg dapagliflozin Capsule 2, fasted) and the reference, Treatment A1 (150 mg AZD9977 (AZD9977 capsule 50 mg + AZD9977 capsule 100 mg) and 10 mg dapagliflozin tablet, fasted). The Cmax was possibly 7% lower than the reference treatment and the C24 value was possibly 23% higher than the reference treatment. See FIG. 4A.

For Treatment C (150 mg AZD9977 + 10 mg dapagliflozin Capsule 1, fed) versus Treatment B (150 mg AZD9977 + 10 mg dapagliflozin Capsule 1, fasted) the AZD9977 AUClast and AUCinf in the fed state were 21% and 18% higher than in the fasted state, respectively. The Cmax was 56% higher in the fed state and the C24 value was 69% lower. See FIG. 4B.

For Treatment E (150 mg AZD9977 + 10 mg dapagliflozin Capsule 2, fed) versus Treatment D (150 mg AZD9977 + 10 mg dapagliflozin Capsule 2, fasted) the AUClast and AUCinf in the fed state were comparable with the values in the fasted state. The Cmax was possibly 31% higher in the fed state and the C24 value was 56% lower. See FIG. 4B.

The AZD9977 AUClast and AUCinf were comparable between Treatment F (150 mg AZD9977 + 10 mg dapagliflozin Capsule 3, fasted) and the reference, Treatment A2 (150 mg AZD9977 [AZD9977 capsule 50 mg + AZD9977 capsule 100 mg] and 10 mg dapagliflozin tablet, fasted). The Cmax was possibly 10% lower than the reference treatment and the C24 value was possibly 11% higher than the reference treatment. See FIG. 5A.

The AZD9977 AUClast and AUCinf were comparable between Treatment G (150 mg AZD9977 + 10 mg dapagliflozin Capsule 4, fasted) and the reference, Treatment A2 (150 mg AZD9977 [AZD9977 capsule 50 mg + AZD9977 capsule 100 mg] and 10 mg dapagliflozin tablet, fasted). The Cmax was possibly 16% lower than the reference treatment and the C24 value was possibly 43% higher than the reference treatment. See FIG. 5A.

For Treatment B (150 mg AZD9977 + 10 mg dapagliflozin Capsule 1, fasted) versus Treatment F (150 mg AZD9977 + 10 mg dapagliflozin Capsule 3, fasted), AZD9977 variant 1, Treatment B AZD9977 exposure (AUClast, AUCinf, Cmax, and C24) was comparable with Treatment F. See FIG. 5B.

For Treatment D (150 mg AZD9977 + 10 mg dapagliflozin Capsule 2, fasted) versus Treatment G (150 mg AZD9977 + 10 mg dapagliflozin Capsule 4, fasted), AZD9977 variant 2, the Treatment D AZD9977 AUClast and AUCinf were comparable with the Treatment G AUClast and AUCinf values. The Treatment D Cmax was possibly 11% higher than the Treatment G Cmax and the Treatment D C24 value was 29% lower than the Treatment G C24 value. See FIG. 5B.

**Plasma Dapagliflozin concentrations following single oral administration of different formulations of AZD9977 and dapagliflozin.**

Geometric mean plasma dapagliflozin concentration versus time curves are presented in FIG. 6A (linear scale) and FIG. 6B (semi-logarithmic scale).

Low between-subject variability in AUCs was observed across all treatments with geometric mean coefficient of variation ranging from approximately 10% to 21%. Low to moderate between-subject variability in Cmax was observed across all treatments with geometric mean coefficient of variation ranging from approximately 17% to 38%.

The dapagliflozin AUClast and AUCinf were comparable between Treatment B (150 mg AZD9977 + 10 mg dapagliflozin Capsule 1, fasted) and the reference, Treatment A1 (150 mg AZD9977 (AZD9977 capsule 50 mg + AZD9977 capsule 100 mg) and 10 mg dapagliflozin tablet, fasted). The Cmax was possibly 11% lower than the reference treatment and the C24 value was possibly 7% higher than the reference treatment. See FIG. 7A.

The dapagliflozin AUClast and AUCinf were comparable between Treatment D (150 mg AZD9977 + 10 mg dapagliflozin Capsule 2, fasted) and the reference, Treatment A1 (150 mg AZD9977 (AZD9977 capsule 50 mg + AZD9977 capsule 100 mg) and 10 mg dapagliflozin tablet, fasted). The Cmax was possibly 9% lower than the reference treatment and the C24 value was 11% higher than the reference treatment. See FIG. 7A.

For Treatment C (150 mg AZD9977 + 10 mg dapagliflozin Capsule 1, fed) versus Treatment B (150 mg AZD9977 + 10 mg dapagliflozin Capsule 1, fasted) the dapagliflozin AUClast and AUCinf in the fed state were comparable with the values observed in the fasted state. The Cmax was 34% lower in the fed state and the C24 value was possibly 12% higher. See FIG. 7B.

For Treatment E (150 mg AZD9977 + 10 mg dapagliflozin Capsule 2, fed) versus Treatment D (150 mg AZD9977 + 10 mg dapagliflozin Capsule 2, fasted) the dapagliflozin AUClast and AUCinf in the fed state were comparable with the values observed in the fasted state. The Cmax was 28% lower in the fed state and the C24 value was comparable with the value observed in the fasted state. See FIG. 7B.

The dapagliflozin AUClast and AUCinf were comparable between Treatment F (150 mg AZD9977 + 10 mg dapagliflozin Capsule 3, fasted) and the reference, Treatment A2 (150 mg AZD9977 (AZD9977 Capsule 50 mg + AZD9977 capsule 100 mg) and 10 mg dapagliflozin tablet, fasted). The Cmax was 22% lower than the Treatment A2 Cmax and the C24 value was 7% higher than the Treatment A2 C24. See FIG. 8A.

For Treatment G (150 mg AZD9977 + 10 mg dapagliflozin Capsule 4, fasted) versus the reference Treatment A2 (150 mg AZD9977 (AZD9977 capsule 50 mg + AZD9977 capsule 100 mg) and 10 mg dapagliflozin tablet, fasted) the AUClast and AUCinf were possibly 8% and 9% higher than the Treatment A2 values, respectively. The Cmax was comparable with the Treatment A2 Cmax and the C24 value was 11% higher than the Treatment A2 C24. See FIG. 8A.

For Treatment H (10 mg dapagliflozin capsule, fasted) versus the reference Treatment A2 (150 mg AZD9977 (AZD9977 capsule 50 mg + AZD9977 capsule 100 mg) and 10 mg dapagliflozin tablet, fasted) the AUClast was 7% higher and the AUCinf was 6% higher than the Treatment A2 values, respectively. The Cmax was 26% lower than the Treatment A2 Cmax and the C24 value was 15% higher than the Treatment A2 C24. See FIG. 8A.

For Treatment B (150 mg AZD9977 + 10 mg dapagliflozin Capsule 1, fasted) versus Treatment G (150 mg AZD9977 + 10 mg dapagliflozin Capsule 4, fasted), dapagliflozin variant 1, the Treatment B dapagliflozin AUClast and AUCinf were 6% lower than the Treatment G AUClast and AUCinf. The Treatment B Cmax was possibly 16% lower than the Treatment G Cmax and the Treatment B C24 value was comparable with the Treatment G C24. See FIG. 8B.

For Treatment D (150 mg AZD9977 + 10 mg dapagliflozin Capsule 2, fasted) versus Treatment F (150 mg AZD9977 + 10 mg dapagliflozin Capsule 3, fasted), dapagliflozin variant 2, the dapagliflozin AUClast and AUCinf were comparable. The Treatment D Cmax was possibly 7% higher than the Treatment F Cmax and the Treatment D C24 value was possibly 8% higher than the Treatment F C24 value. See FIG. 8B.

**Pharmacokinetic Conclusions.**

Exposure to AZD9977 and dapagliflozin from all four capsules (Treatment B, D, F, G) under fasted conditions was generally comparable to the respective reference treatment (A1 and A2) in terms of AUClast and AUCinf. However, there was a trend towards a lower Cmax and a higher C24 than observed for the reference treatment.

Administration of Capsule 1 and Capsule 2 with food resulted in a slightly delayed tmax for AZD9977. For Treatment C (Capsule 1, fed) the AUClast, AUCinf and Cmax of AZD9977 values were higher than that observed for Treatment B (Capsule 1, fasted), with the C24 value being lower in the fed state. For Treatment E (Capsule 2, fed) the AUClast, AUCinf of AZD9977 values were comparable to Treatment D (Capsule 2, fasted), with Cmax being possibly higher and C24 value being lower in fed than fasted state. Overall, the magnitude of the impact of food on AZD9977 PK appeared to be lower in Capsule 2 than in Capsule 1.

For dapagliflozin in Treatment C (Capsule 1, fed) and Treatment E (Capsule 2, fed), the tmax in the fed state was slightly later than the fasted tmax, but exposure to dapagliflozin in terms of AUClast and AUCinf was comparable between

fed and fasted states. For Treatment C the Cmax was lower than the Treatment B Cmax and the C24 was higher than the Treatment B C24. For Treatment E the Cmax was lower than the Treatment D Cmax and the C24 values were comparable.

Exposure to AZD9977 variant 1 from Capsule 1 (Treatment B) was comparable with that from Capsule 3 (Treatment F) in terms of AUClast, AUCinf, Cmax and C24. Exposure to AZD9977 variant 2 from Capsule 2 (Treatment D) was comparable with that from Capsule 4 (Treatment G) in terms of AUClast and AUCinf. The Treatment D Cmax was possibly 11% higher than the Treatment G Cmax and the Treatment D C24 value was 29% lower than the Treatment G C24 value.

Exposure to dapagliflozin variant 1 from Capsule 1 (Treatment B) was 6% lower than that from Capsule 4 (Treatment G) in terms of AUClast and AUCinf. The Treatment B Cmax was possibly 16% lower than the Treatment G Cmax and the C24 values were comparable.

Exposure to dapagliflozin variant 2 from Capsule 2 (Treatment D) was comparable with that from Capsule 3 (Treatment F) in terms of AUClast and AUCinf. The Treatment D Cmax and C24 values were possibly 7-8% higher than the Treatment F values.

For AZD9977 there were common trends to all of the capsule formulations in the fasted state; the AUClast and AUCinf values of capsules (Treatment B, C, D, E, F, and G) were generally comparable with those observed for the respective reference treatment (A1 or A2) but Cmax tended to be lower than the reference treatment whilst C24 tended to be higher. A similar trend was observed for dapagliflozin, with the slight exception of Treatment G where the AUClast and AUCinf values were slightly higher than the reference treatment, Cmax was comparable with the reference treatment and C24 tended to be slightly lower.

Administration of Capsule 1 and Capsule 2 with food resulted in a slightly delayed tmax for AZD9977. For Treatment C (Capsule 1, fed) the AUClast, AUCinf and Cmax values were notably higher than observed for Treatment B (Capsule 1, fasted), with the C24 value being notably lower in the fed state. For Treatment E (Capsule 2, fed) the effect was less pronounced with comparable exposure to AZD9977 in the fasted condition in terms of AUClast and AUCinf, although the Cmax appeared higher and C24 lower in the fed state. For dapagliflozin in Treatment C and Treatment E, the tmax in the fed state was slightly later than the fasted tmax but exposure to dapagliflozin in terms of AUClast and AUCinf was comparable between fed and fasted states. The Cmax for dapagliflozin was lower in the fed state for both Capsule 1 and Capsule 2, C24 was slightly higher for Capsule 1 but was comparable for Capsule 2.

The effect of food on the pharmacokinetics of AZD9977 and dapagliflozin is likely to have no clinical relevance and the fixed-dose combination of AZD9977 /Dapagliflozin is anticipated to be taken without dietary instructions.

AZD9977 variant 1 was dosed as Treatment B (Capsule 1) and Treatment F (Capsule 3) in combination with different variants of dapagliflozin. Exposure to AZD9977 variant 1 from Capsule 1 was comparable with that from Capsule 3 in terms of AUClast, AUCinf, Cmax and C24. AZD9977 variant 2 was dosed as Treatment D (Capsule 2) and Treatment G (Capsule 4). Exposure to AZD9977 variant 2 from Capsule 2 was comparable with that from Capsule 4 in terms of AUClast and AUCinf, although the Cmax values seemed to be possibly higher for Capsule 2 and the C24 was possibly lower for Capsule 2.

The same dapagliflozin variant was dosed in different capsules, variant 1 was dosed as Treatment B (Capsule 1) and Treatment G (Capsule 4) and variant 2 was dosed as Treatment D (Capsule 2) and Treatment F (Capsule 3). Exposure to variant 1 of dapagliflozin was possibly lower for Capsule 1 in terms of AUClast and AUCinf whilst exposure to variant 2 of dapagliflozin from Capsule 2 was comparable with that from Capsule 3 for AUClast and AUCinf. For variant 1, the Cmax for Treatment B (Capsule 1) was possibly lower than that observed for Treatment G (Capsule 4), with the C24 being comparable with that observed for Treatment G (Capsule 4). For variant 2, the Cmax and C24 for Treatment D (Capsule 2) was possibly higher than that observed for Treatment F (Capsule 3).

**EXAMPLE 4. Phase 3 Formulation of AZD9977 and Dapagliflozin Capsules**

**Immediate Release (IR) AZD9977 pellets:**

**Preparation of AZD9977 spray coating suspension (A1 suspension).** 5.1 kg of polyvinylpyrrolidone (povidone; PVP) K30 (BASF) was added to 130.9 kg of purified water and left stirring for at least 0.5 h (until clear solution). 34 kg of AZD9977 (STA WuXi) was added to the PVPK30/water solution to produce a 23 % (w/w) AZD9977 coating suspension that was left stirring overnight at RT. The suspension was particle size reduced using a co-ball mill.

**Spray coating of 600 mg/g AZD9977 IR pellets (A1).** 17 kg of microcrystalline cellulose (MCC) cores (VIVAPUR® 100, JRS Pharma) was loaded into a Wurster fluid bed spray coater (GPCG30). The cores were coated with 165 kg of the AZD9977 coating suspension at the conditions summarized in Table 2 to produce 54.2 kg of AZD9977 IR pellets with 601 mg/g drug load.

**Immediate Release (IR) Dapa pellets:**

**Preparation of Dapa spray coating suspension.** 0.9 kg of Hydroxypropylcellulose (HPC SSL from Nisso) was added to 67.5 kg of purified water and left stirring for at least 0.5h (until clear solution). 14.85 kg talc (Imerys) and 6.75 kg Dapagliflozin (Dottikon) was added to the HPC solution to produce a 25% (w/w) Dapa coating suspension. The suspension was left stirring overnight at RT.

**Spray coating of 105 mg/g Dapa IR pellets.** 30 kg of Sugar spheres (NP) cores (Vivapharm®, JRS Pharma) was loaded into a Wurster fluid bed spray coater (GPCG30). The cores were coated with 90 kg of the Dapa coating suspension at the condition summarized in Table 2 to produce 49.5 kg of Dapa IR pellets with 107 mg/g drug load.

**Table 2. Spray Coating Process Parameters Phase 3.**

| Process Parameter | Dapagliflozin Pellet | AZD9977 Pellet A1 | Unit |
|---|---|---|---|
| Inlet fluidising gas temp | 97-99 | 95-105 | [°C] |
| Outlet fluidising gas temp | 44-63 | 45-50 | [°C] |
| Fluidising gas flow | Approx. 1254 | Approx. 1045 | [$Nm^3/h$] |
| Water amount in fluidising gas | 5.8 | 6.0 | [g/kg] |
| Atomizer flow | 70 | 47-53 | [$Nm^3/h$] |
| Atomizer pressure | 4 | 2.5-2.7 | [bar] |
| Average spray rate | 500 | 410 | [g/min] |

**Filling of Pellets into Capsules**

**Lubrication:**

**AZD9977:** 49.9 kg of the AZD9977 pellets were mixed with 0.1 kg of sodium stearyl fumarate (SSF PRUV, Moehs) in a double cone blender 200L for 10 min/15 rpm to produce 50.0 kg of lubricated AZD9977 pellets.

**Dapagliflozin:** 99.8 kg of the dapagliflozin pellets were mixed with 0.2 kg of sodium stearyl fumarate (SSF PRUV, Moehs) in a double cone blender 200L for 10 min/15 rpm to produce 100.0 kg of lubricated dapagliflozin pellets.

**FDC Capsules:**

**Dose 50 mg AZD9977 and 10 mg Dapagliflozin.** 50.0 kg of lubricated AZD9977 pellets and 57.5 kg of lubricated dapagliflozin pellets were filled into 600 000 white, size 1EL gelatin capsules (Capsugel) with an average fill weight of 83.4 mg AZD9977 pellets and 95.9 mg dapagliflozin pellets using an MG Futura encapsulator.

**Dose 150 mg AZD9977 and 10 mg Dapagliflozin.** 50.0 kg of lubricated AZD9977 pellets and 19.2 kg of lubricated dapagliflozin pellets were filled into 200 000 white, size 1EL gelatin capsules (Capsugel) with an average fill weight of 250 mg AZD9977 pellets and 95.9 mg dapagliflozin pellets using an MG Futura encapsulator.

**Claims**

**1.** A pharmaceutical composition comprising:

(a) one or more of a first pellet comprising

i. a first core, and
ii. a first coating on the first core, wherein the first coating comprises a mineralocorticoid receptor (MR) modulator and a first binder; and

(b) one or more of a second pellet comprising

i. a second core, and
ii. a second coating on the second core, wherein the second coating comprises an SGLT2 inhibitor, wherein the SGLT2 inhibitor is about 5% to about 20% by weight of the second pellet,

wherein the composition comprises about 20% to about 50% by weight of the mineralocorticoid receptor (MR) modulator; and about 1% to about 10% by weight of the SGLT2 inhibitor.

**2.** The pharmaceutical composition of claim 1, wherein the MR modulator comprises a compound of Formula I:

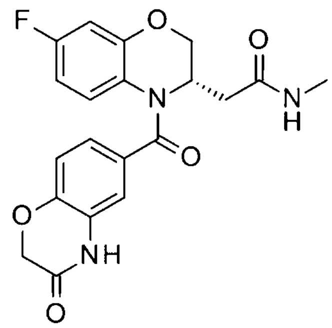

Formula I (AZD9977 or balcinrenone).

**3.** The pharmaceutical composition of claim 1 or 2, wherein the composition comprises about 25% to about 45% by weight of the MR modulator.

**4.** The pharmaceutical composition of any one of claims 1 to 3, wherein the composition comprises either 25% to about 30% by weight of the MR modulator or about 40% or about 45% by weight of the MR modulator.

**5.** The pharmaceutical composition of any one of claims 1 to 4, wherein the first binder comprises povidone.

**6.** The pharmaceutical composition of claim 5, wherein the composition comprises about 1% to about 10% by weight povidone.

**7.** The pharmaceutical composition of claim 6, wherein the composition comprises about 4% to about 5% by weight povidone or about 6% to about 7% by weight povidone.

**8.** The pharmaceutical composition of any one of claims 1 to 4, wherein the first binder comprises povidone and hypromellose.

**9.** The pharmaceutical composition of claim 8, wherein the composition comprises about 4% to about 6% by weight povidone.

**10.** The pharmaceutical composition of claim 8 or 9, wherein the composition comprises about 0.5% to about 3% by weight hypromellose.

**11.** The pharmaceutical composition of any one of claims 8 to 10, wherein the composition comprises about 5% by weight povidone and about 1% by weight hypromellose.

**12.** The pharmaceutical composition of any one of claims 1 to 11, wherein the first coating further comprises a first lubricant.

**13.** The pharmaceutical composition of claim 12, wherein the first lubricant comprises sodium stearyl fumarate.

**14.** The pharmaceutical composition of claim 12 or 13, wherein the first lubricant is about 0.01% to about 0.5% by weight of the composition.

**15.** The pharmaceutical composition of any one of claims 1 to 14, wherein the first core comprises microcrystalline cellulose.

**16.** The pharmaceutical composition of claim 15, wherein the first core is about 10% to about 30% by weight of the composition.

**17.** The pharmaceutical composition of any one of claims 1 to 16, wherein the SGLT2 inhibitor comprises dapagliflozin.

**18.** The pharmaceutical composition of claim 17, wherein the SGLT2 inhibitor comprises either non-crystalline dapagliflozin or crystalline dapagliflozin.

**19.** The pharmaceutical composition of any one of claims 1 to 18, wherein the composition comprises about 2% to about 8% by weight of the SGLT2 inhibitor.

**20.** The pharmaceutical composition of any one of claims 1 to 19, wherein the composition comprises about 2.5% to about 4% of the SGLT2 inhibitor.

**21.** The pharmaceutical composition of any one of claims 1 to 20, wherein the SGLT2 inhibitor is about 5% to about 15% by weight of the second pellet.

**22.** The pharmaceutical composition of claim 21, wherein the SGLT2 inhibitor is about 7% to about 13% by weight of the second pellet.

**23.** The pharmaceutical composition of claim 22, wherein the SGLT2 inhibitor is about 8% to about 12% by weight of the second pellet.

**24.** The pharmaceutical composition of any one of claims 1 to 20, wherein the SGLT2 inhibitor is about 10% to about 15% by weight of the second pellet.

**25.** The pharmaceutical composition of claim 24, wherein the SGLT2 inhibitor is about 12% to about 14% by weight of the second pellet.

**26.** The pharmaceutical composition of claim 25, wherein the SGLT2 inhibitor is about 12.8% by weight of the second pellet.

**27.** The pharmaceutical composition of any one of claims 1 to 26, wherein the second coating further comprises a second binder, an anti-tacking agent, and a second lubricant.

**28.** The pharmaceutical composition of claim 27, wherein the second binder comprises hydroxypropyl cellulose.

**29.** The pharmaceutical composition of claim 27 or 28, wherein the second binder is about 0.1% to about 5% by weight of the composition.

**30.** The pharmaceutical composition of any one of claims 27 to 29, wherein the anti-tacking agent comprises talc.

**31.** The pharmaceutical composition of any one of claims 27 to 30, wherein the anti-tacking agent is about 1% to about 20% by weight of the composition.

**32.** The pharmaceutical composition of any one of claims 27 to 31, wherein the second lubricant comprises sodium stearyl fumarate.

**33.** The pharmaceutical composition of any one of claims 27 to 32, wherein the second lubricant is about 0.01% to about 1% by weight of the composition.

**34.** The pharmaceutical composition of any one of claims 1 to 33, wherein the second core comprises a sugar, a starch, or combination thereof.

**35.** The pharmaceutical composition of claim 34, wherein the second core is about 10% to about 40% by weight of the composition.

**36.** A pharmaceutical composition in the form of a capsule, comprising:

(a) one or more of a first pellet comprising:

i. a first core comprising a microcrystalline cellulose core, wherein the first core is about 5% to about 25% by weight of the capsule;

ii. a first coating comprising (A) an MR modulator, wherein the MR modulator is AZD9977, and wherein the AZD9977 is about 10% to about 45% by weight of the capsule; (B) povidone, wherein the povidone is about 1% to about 10% by weight of the capsule; and (C) sodium stearyl fumarate, wherein the sodium stearyl fumarate is about 0.01% to about 1% by weight of the capsule; and

(b) one or more of a second pellet comprising:

i. a second core comprising a sugar sphere, wherein the sugar sphere is about 5% to about 30% by weight of the capsule;
ii. a second coating comprising (A) an SGLT2 inhibitor, wherein the SGLT2 inhibitor is dapagliflozin propanediol, and wherein the dapagliflozin propanediol is about 1% to about 10% by weight of the capsule and about 5% to about 20% by weight of the second pellet; (B) hydroxypropyl cellulose, wherein the hydroxypropyl cellulose is about 0.1% to about 1% by weight of the capsule; (C) talc, wherein the talc is about 1% to about 15% by weight of the capsule; and (D) sodium stearyl fumarate, wherein the sodium stearyl fumarate is about 0.01% to about 1% by weight of the capsule.

**37.** A pharmaceutical composition in the form of a capsule, comprising:

(a) one or more of a first pellet comprising:

i. a first core comprising a microcrystalline cellulose core, wherein the first core is about 5% to about 25% by weight of the capsule;
ii. a first coating comprising (A) an MR modulator, wherein the MR modulator is AZD9977, and wherein the AZD9977 is about 15% to about 40% by weight of the capsule, (B) povidone, wherein the povidone is about 2% to about 8% by weight of the capsule; and (C) sodium stearyl fumarate, wherein the sodium stearyl fumarate is about 0.1% to about 0.5% by weight of the capsule; and

(b) one or more of a second pellet comprising:

i. a second core comprising a sugar sphere, wherein the second core is about 10% to about 25% by weight of the capsule;
ii. a second coating comprising (A) an SGLT2 inhibitor, wherein the SGLT2 inhibitor is dapagliflozin propanediol, and wherein the dapagliflozin propanediol is about 2% to about 5% by weight of the capsule and about 5% to about 20% by weight of the second pellet, (B) hydroxypropyl cellulose, wherein the hydroxypropyl cellulose is about 0.2% to about 0.8% by weight of the capsule; (C) talc, wherein the talc is about 5% to about 12% by weight of the capsule; and (D) sodium stearyl fumarate, wherein the sodium stearyl fumarate is about 0.03% to about 0.1% by weight of the capsule.

**38.** The pharmaceutical composition of claim 36 or 37, wherein the capsule comprises either about 50 mg or about 150 mg AZD9977.

**39.** The pharmaceutical composition of any one of claims 36 to 38, wherein the capsule comprises about 10 mg dapagliflozin propanediol.

**40.** An oral dosage form comprising the pharmaceutical composition of any one of claims 1 to 35.

**41.** The oral dosage form of claim 40, wherein the oral dosage form is a capsule.

**42.** The oral dosage form of claim 40 or 41, wherein the pharmaceutical composition in the dosage form comprises either about 50 mg or about 150 mg AZD9977.

**43.** The oral dosage form of any one of claims 40 to 42, wherein the pharmaceutical composition in the dosage form comprises about 10 mg dapagliflozin propanediol.

**44.** The pharmaceutical composition of any one of claims 1 to 39, or the oral dosage form of claims 40 to 43, wherein at least 80% of the MR modulator is released within 30 minutes.

**45.** The pharmaceutical composition of any one of claims 1 to 39, or the oral dosage form of claims 40 to 44, wherein at

least 80% of the SGLT2 inhibitor is released within 30 minutes.

**46.** A method of treating heart failure in a subject in need thereof, comprising administering the pharmaceutical composition of any one of claims 1 to 39 or the oral dosage form of any one of claims 40 to 45 to the subject.

**47.** The method of claim 46, wherein a daily dose of about either 50 mg or about 150 mg of the MR modulator and a daily dose of about 10 mg of the SGLT2 inhibitor are administered.

**48.** The method of claim 46 or 47, wherein the administering is once daily.

**49.** The method of any one of claims 46 to 48, wherein the pharmaceutical composition or oral dosage form is administered to the subject in a fasted state.

**50.** The method of any one of claims 46 to 49, wherein an $AUC_{last}$ and $AUC_{inf}$ of the subject following the administering are within 10% of an $AUC_{last}$ and $AUC_{inf}$ of the subject administered with separate dosage forms of the MR modulator and the SGLT2 inhibitor.

**51.** A method of treating chronic kidney disease in a subject in need thereof, comprising administering the pharmaceutical composition of any one of claims 1 to 39 or the oral dosage form of any one of claims 40 to 45 to the subject.

**52.** The method of claim 51, wherein a daily dose of about either 50 mg or about 150 mg of the MR modulator and a daily dose of about 10 mg of the SGLT2 inhibitor are administered.

**53.** The method of claim 51 or 52, wherein the administering is once daily.

**54.** The method of any one of claims 51 to 53, wherein the pharmaceutical composition or oral dosage form is administered to the subject in a fasted state.

**55.** The method of any one of claims 51 to 54, wherein an $AUC_{last}$ and $AUC_{inf}$ of the subject following the administering are within 10% of an $AUC_{last}$ and $AUC_{inf}$ of the subject administered with separate dosage forms of the MR modulator and the SGLT2 inhibitor.

FIG. 1A

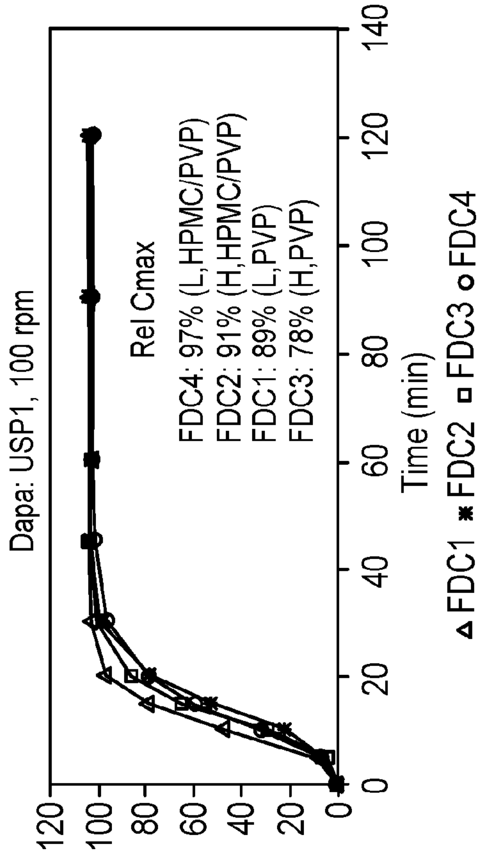


FIG. 1B

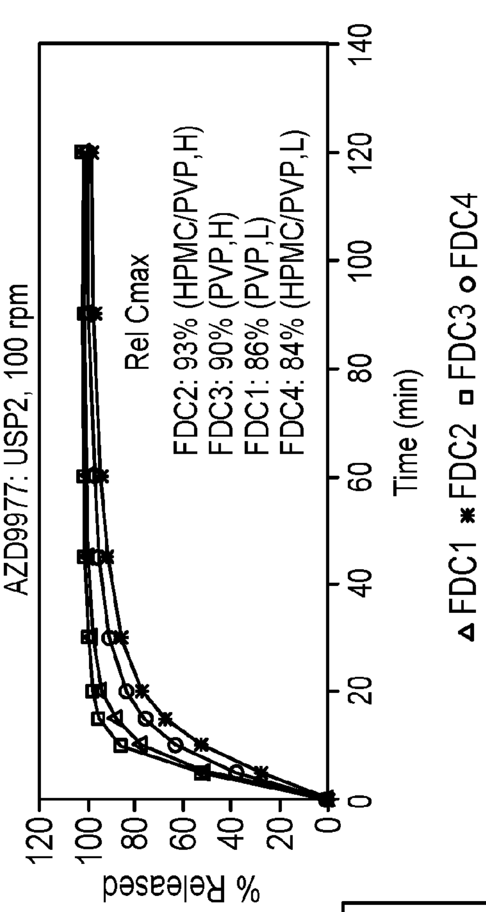


FIG. 1C

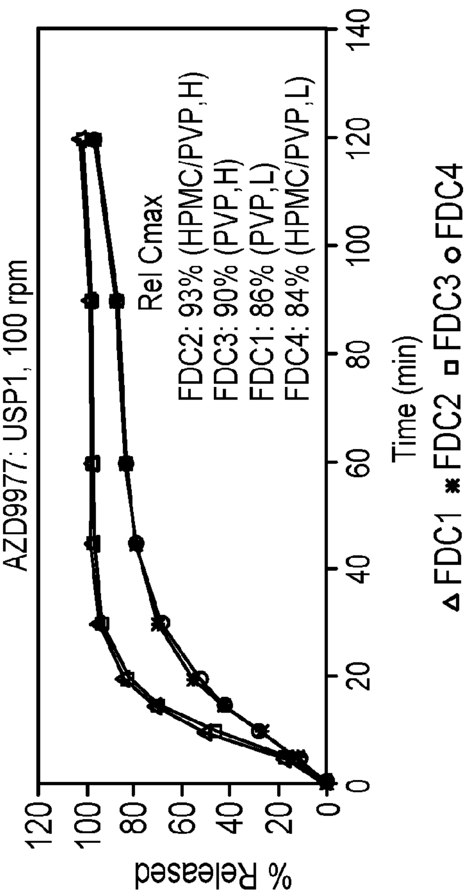

FIG. 2A

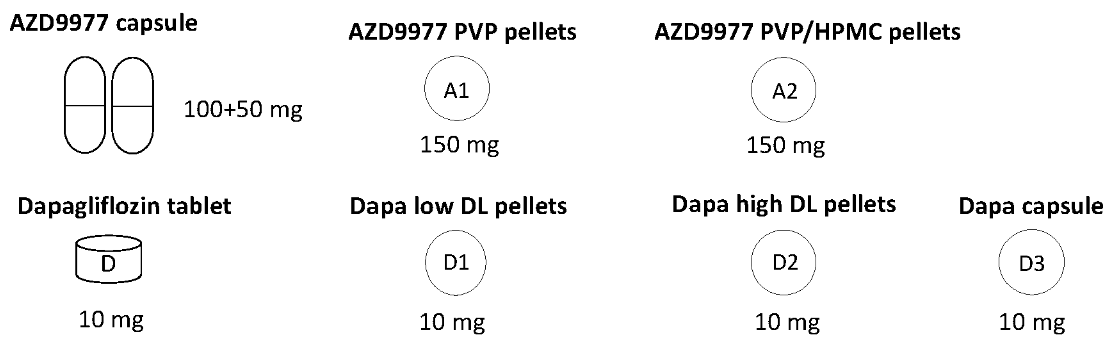
AZD9977 capsule
100+50 mg
AZD9977 PVP pellets
A1
150 mg
AZD9977 PVP/HPMC pellets
A2
150 mg
Dapagliflozin tablet
D
10 mg
Dapa low DL pellets
D1
10 mg
Dapa high DL pellets
D2
10 mg
Dapa capsule
D3
10 mg

FIG. 2B

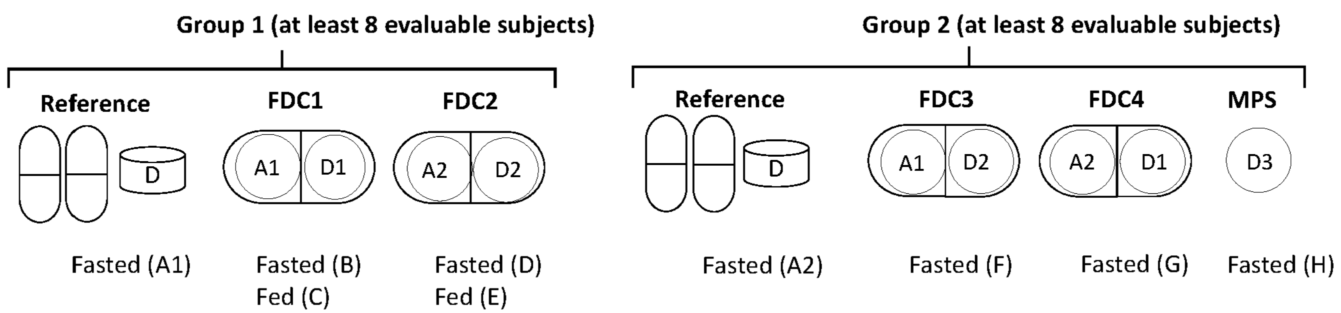
Group 1 (at least 8 evaluable subjects)
Reference
D
Fasted (A1)
FDC1
A1
D1
Fasted (B)
Fed (C)
FDC2
A2
D2
Fasted (D)
Fed (E)
Group 2 (at least 8 evaluable subjects)
Reference
D
Fasted (A2)
FDC3
A1
D2
Fasted (F)
FDC4
A2
D1
Fasted (G)
MPS
D3
Fasted (H)

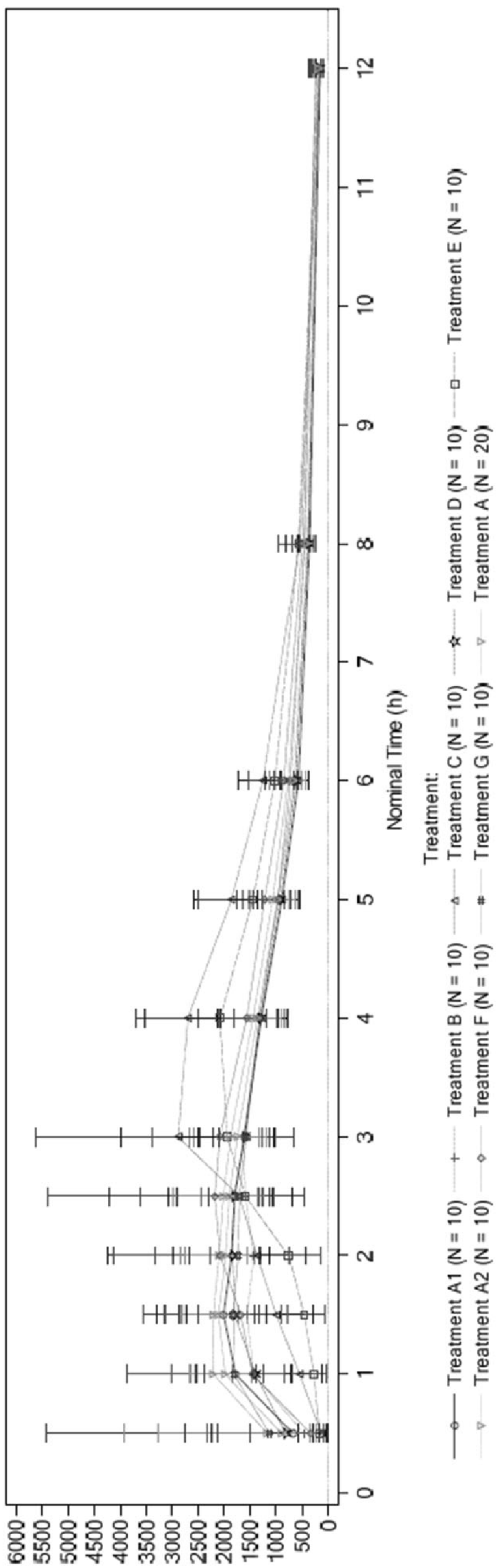
Geometric Mean Plasma Concentrations (nmol/L)
6000
5500
5000
4500
4000
3500
3000
2500
2000
1500
1000
500
0
0
1
2
3
4
5
6
7
8
9
10
11
12
Nominal Time (h)
Treatment:
Treatment A1 (N = 10)
Treatment B (N = 10)
Treatment C (N = 10)
Treatment D (N = 10)
Treatment E (N = 10)
Treatment A2 (N = 10)
Treatment F (N = 10)
Treatment G (N = 10)
Treatment A (N = 20)

FIG. 3A

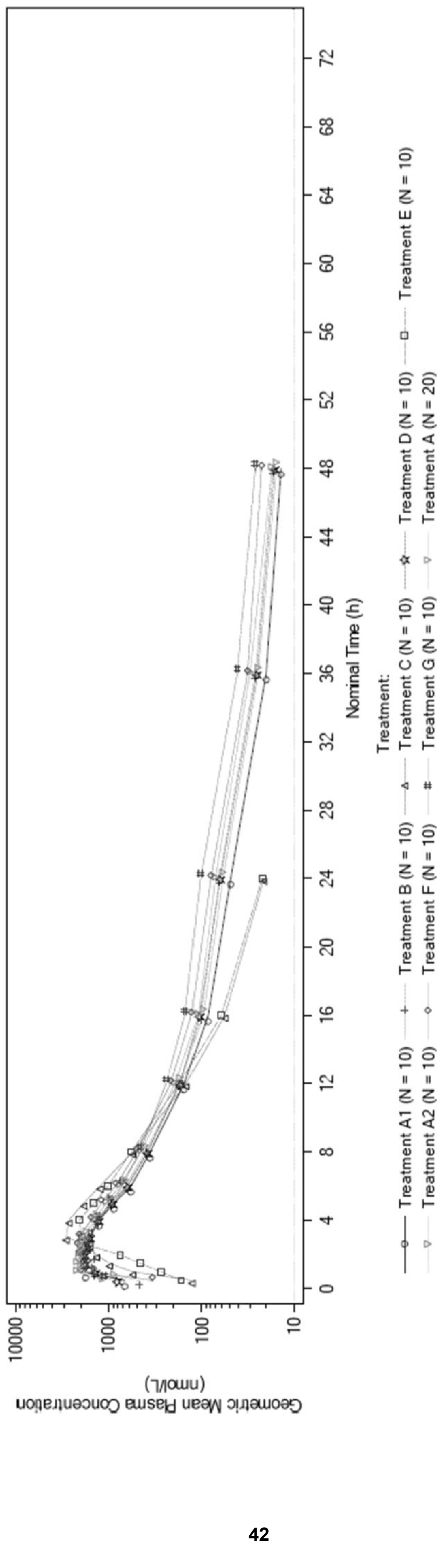
Geometric Mean Plasma Concentration (nmol/L)
10000
1000
100
10
0
4
8
12
16
20
24
28
32
36
40
44
48
52
56
60
64
68
72
Nominal Time (h)
Treatment:
Treatment A1 (N = 10)
Treatment B (N = 10)
Treatment C (N = 10)
Treatment D (N = 10)
Treatment E (N = 10)
Treatment A2 (N = 10)
Treatment F (N = 10)
Treatment G (N = 10)
Treatment A (N = 20)

FIG. 3B

FIG. 4A

| Parameter (Units) | Treatment | N | Geometric LS Mean | Comparison of Treatment Groups |
|---|---|---|---|---|
| | | | | Geometric Mean Ratio (90% CI) |
| AUCinf (h*nmol/L) | Treatment B | 10 | 12600 | 98.10 (89.26, 107.83) |
| | Treatment A1 | 10 | 12840 | |
| | Treatment D | 10 | 13460 | 104.82 (96.08, 114.36) |
| | Treatment A1 | 10 | 12840 | |
| AUClast (h*nmol/L) | Treatment B | 10 | 12130 | 96.41 (86.59, 107.34) |
| | Treatment A1 | 10 | 12580 | |
| | Treatment D | 10 | 13000 | 103.29 (93.55, 114.03) |
| | Treatment A1 | 10 | 12580 | |
| Cmax (nmol/L) | Treatment B | 10 | 2272 | 86.20 (65.92, 112.72) |
| | Treatment A1 | 10 | 2636 | |
| | Treatment D | 10 | 2447 | 92.82 (72.49, 118.85) |
| | Treatment A1 | 10 | 2636 | |
| C24 (nmol/L) | Treatment B | 10 | 67.94 | 148.86 (101.28, 218.77) |
| | Treatment A1 | 10 | 45.64 | |
| | Treatment D | 10 | 56.22 | 123.17 (86.40, 175.60) |
| | Treatment A1 | 10 | 45.64 | |

Treatment A1 150 mg AZD9977 (AZD9977 capsule 50 mg + AZD9977 capsule 100 mg) and 10 mg dapagliflozin tablet, fasted in Group 1. Treatment B 150 mg AZD9977 + 10 mg dapagliflozin Capsule 1, fasted. Treatment D 150 mg AZD9977 + 10 mg dapagliflozin Capsule 2, fasted. CI Confidence interval; LS Least-squares; N Number of subjects per treatment group. Geometric mean ratio and corresponding 90% CI are back-transformed.

Result based on a linear mixed effect analysis of variance (ANOVA) of log transformed PK parameter with sequence, period, treatment as fixed-effect and subject nested within sequence as random effect.

FIG. 4B

| Parameter (Units) | Treatment | N | Geometric LS Mean | Comparison of Treatment Groups |
|---|---|---|---|---|
| | | | | Geometric Mean Ratio (90% CI) |
| AUCinf (h*nmol/L) | Treatment C | 10 | 14870 | 118.07 (108.27, 128.76) |
| | Treatment B | 10 | 12600 | |
| | Treatment E | 10 | 13750 | 102.17 (92.25, 113.16) |
| | Treatment D | 10 | 13460 | |
| AUClast (h*nmol/L) | Treatment C | 10 | 14710 | 121.24 (109.87, 133.79) |
| | Treatment B | 10 | 12130 | |
| | Treatment E | 10 | 13510 | 103.96 (92.57, 116.76) |
| | Treatment D | 10 | 13000 | |
| Cmax (nmol/L) | Treatment C | 10 | 3551 | 156.28 (122.20, 199.86) |
| | Treatment B | 10 | 2272 | |
| | Treatment E | 10 | 3195 | 130.59 (97.71, 174.53) |
| | Treatment D | 10 | 2447 | |
| C24 (nmol/L) | Treatment C | 10 | 20.88 | 30.73 (21.60, 43.72) |
| | Treatment B | 10 | 67.94 | |
| | Treatment E | 9 | 24.76 | 44.04 (28.71, 37.56) |
| | Treatment D | 10 | 56.22 | |

Treatment B 150 mg AZD9977 + 10 mg dapagliflozin Capsule 1, fasted. Treatment C 150 mg AZD9977 + 10 mg dapagliflozin Capsule 1, fed. Treatment D 150 mg AZD9977 + 10 mg dapagliflozin Capsule 2, fasted. Treatment E 150 mg AZD9977 + 10 mg dapagliflozin Capsule 2, fed. CI Confidence interval; LS Least-squares; N Number of subjects per treatment group. Geometric mean ratio and corresponding 90% CI are back-transformed.

Result based on a linear mixed effect analysis of variance (ANOVA) of log transformed PK parameter with sequence, period, treatment as fixed-effect and subject nested within sequence as random effect.

FIG. 5A

| Parameter (Units) | Treatment | N | Geometric LS Mean | Comparison of Treatment Groups |
|---|---|---|---|---|
| | | | | Geometric Mean Ratio (90% CI) |
| AUCinf (h*nmol/L) | Treatment F | 10 | 15400 | 104.31 (98.64, 110.29) |
| | Treatment A2 | 10 | 14760 | |
| | Treatment G | 10 | 15510 | 105.10 (99.35, 111.18) |
| | Treatment A2 | 10 | 14760 | |
| AUClast (h*nmol/L) | Treatment F | 10 | 14770 | 102.50 (95.22, 110.33) |
| | Treatment A2 | 10 | 14410 | |
| | Treatment G | 10 | 14900 | 103.43 (96.03, 111.40) |
| | Treatment A2 | 10 | 14410 | |
| Cmax (nmol/L) | Treatment F | 10 | 2552 | 89.69 (70.83, 113.57) |
| | Treatment A2 | 10 | 2845 | |
| | Treatment G | 10 | 2399 | 84.32 (66.46, 106.97) |
| | Treatment A2 | 10 | 2845 | |
| C24 (nmol/L) | Treatment F | 10 | 80.03 | 110.97 (64.09, 192.14) |
| | Treatment A2 | 10 | 72.12 | |
| | Treatment G | 10 | 103.0 | 142.88 (82.17, 248.46) |
| | Treatment A2 | 10 | 72.12 | |

Treatment A2 150 mg AZD9977 (AZD9977 capsule 50 mg + AZD9977 capsule 100 mg) and 10 mg dapagliflozin tablet, fasted in Group 2. Treatment F 150 mg AZD9977 + 10 mg dapagliflozin Capsule 3, fasted. Treatment G 150 mg AZD9977 + 10 mg dapagliflozin Capsule 4, fasted. CI Confidence interval;
LS Least-squares; N Number of subjects per treatment group. Geometric mean ratio and corresponding 90% CI are back-transformed.
Result based on a linear mixed effect analysis of variance (ANOVA) of log transformed PK parameter with sequence, period, treatment as fixed-effect and subject nested within sequence as random effect.

FIG. 5B

| Parameter (Units) | Treatment | N | Geometric LS Mean | Comparison of Treatment Groups Geometric Mean Ratio (90% CI) |
|---|---|---|---|---|
| AUCinf (h*nmol/L) | Treatment B | 10 | 13300 | 97.10 (88.43, 106.63) |
| | Treatment F | 10 | 13700 | |
| | Treatment D | 10 | 14140 | 102.97 (93.39, 113.54) |
| | Treatment G | 10 | 13740 | |
| AUClast (h*nmol/L) | Treatment B | 10 | 12800 | 97.33 (87.55, 108.20) |
| | Treatment F | 10 | 13150 | |
| | Treatment D | 10 | 13580 | 102.82 (92.03, 114.87) |
| | Treatment G | 10 | 13210 | |
| Cmax (nmol/L) | Treatment B | 10 | 2366 | 101.64 (79.49, 129.97) |
| | Treatment F | 10 | 2328 | |
| | Treatment D | 10 | 2429 | 111.17 (85.56, 144.45) |
| | Treatment G | 10 | 2185 | |
| C24 (nmol/L) | Treatment B | 10 | 73.60 | 99.02 (64.74, 151.47) |
| | Treatment F | 10 | 74.32 | |
| | Treatment D | 10 | 67.35 | 70.72 (44.92, 111.35) |
| | Treatment G | 10 | 95.23 | |

Treatment B 150 mg AZD9977 + 10 mg dapagliflozin Capsule 1, fasted. Treatment D 150 mg AZD9977 + 10 mg dapagliflozin Capsule 2, fasted. Treatment F 150 mg AZD9977 + 10 mg dapagliflozin Capsule 3, fasted. Treatment G 150 mg AZD9977 + 10 mg dapagliflozin Capsule 4, fasted. CI Confidence interval; LS Least-squares; N Number of subjects per treatment group.

Result based on a linear mixed effect analysis of variance (ANOVA) of log transformed PK parameter with period, treatment as fixed-effect and subject nested within sequence as random effect and adjusting for the values of reference Treatment A. Geometric mean ratio and corresponding 90% CI are back-transformed.

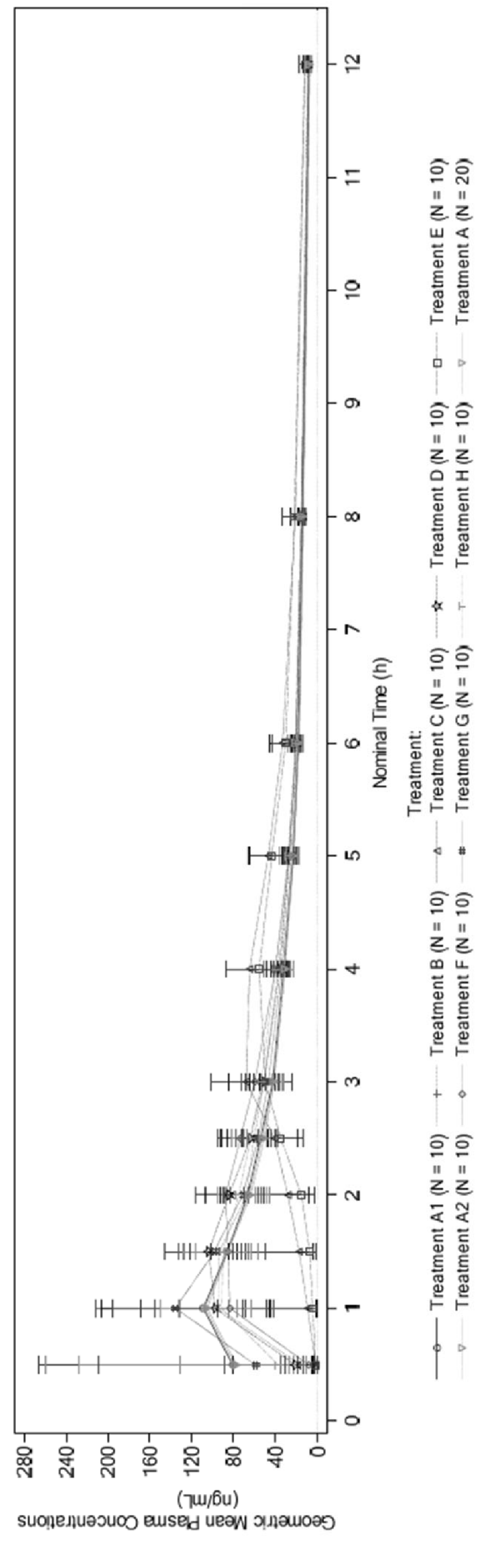
Geometric Mean Plasma Concentrations (ng/mL)
0
40
80
120
160
200
240
280
Nominal Time (h)
0
1
2
3
4
5
6
7
8
9
10
11
12
Treatment:
Treatment A1 (N = 10)
Treatment B (N = 10)
Treatment C (N = 10)
Treatment D (N = 10)
Treatment E (N = 10)
Treatment A2 (N = 10)
Treatment F (N = 10)
Treatment G (N = 10)
Treatment H (N = 10)
Treatment A (N = 20)

FIG. 6A

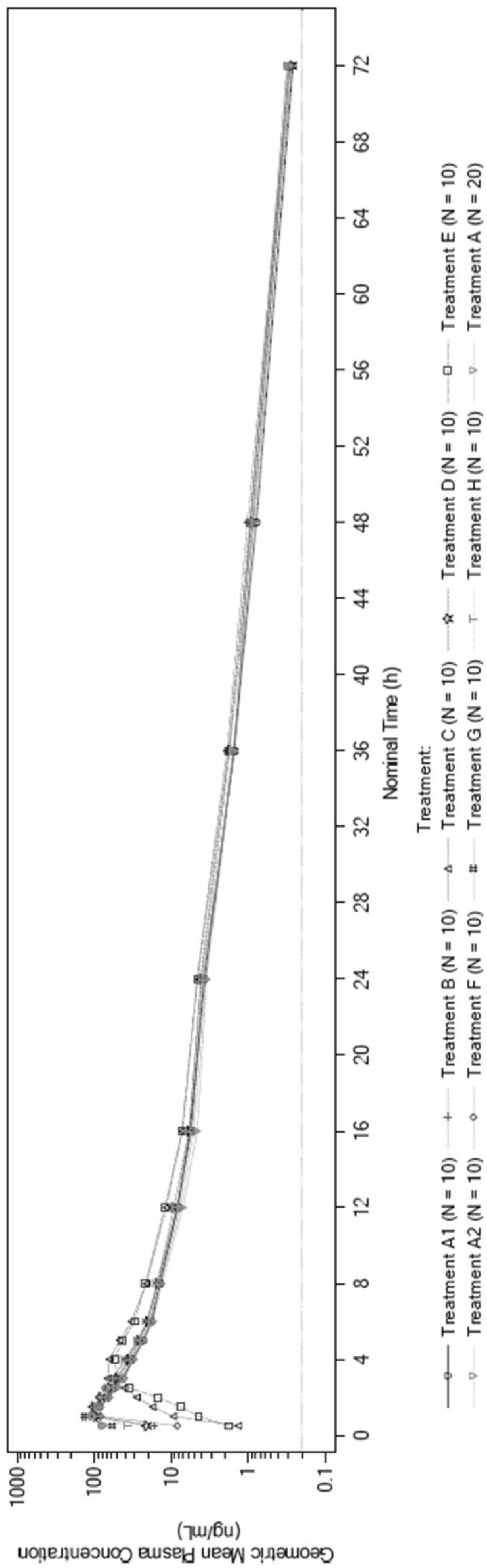

Geometric Mean Plasma Concentration (ng/mL)
1000
100
10
1
0.1
0
4
8
12
16
20
24
28
32
36
40
44
48
52
56
60
64
68
72
Nominal Time (h)
Treatment:
Treatment A1 (N = 10)
Treatment B (N = 10)
Treatment C (N = 10)
Treatment D (N = 10)
Treatment E (N = 10)
Treatment A2 (N = 10)
Treatment F (N = 10)
Treatment G (N = 10)
Treatment H (N = 10)
Treatment A (N = 20)


FIG. 6B

FIG. 7A

| Parameter (Units) | Treatment | N | Geometric LS Mean | Comparison of Treatment Groups Geometric Mean Ratio (90% CI) |
|---|---|---|---|---|
| AUCinf (h*ng/mL) | Treatment B | 10 | 517.2 | 100.17 (96.25, 104.25) |
| | Treatment A1 | 10 | 516.3 | |
| | Treatment D | 10 | 531.5 | 102.95 (99.22, 106.81) |
| | Treatment A1 | 10 | 516.3 | |
| AUClast (h*ng/mL) | Treatment B | 10 | 505.9 | 99.81 (95.98, 103.80) |
| | Treatment A1 | 10 | 506.8 | |
| | Treatment D | 10 | 523.2 | 103.24 (99.57, 107.04) |
| | Treatment A1 | 10 | 506.8 | |
| Cmax (ng/mL) | Treatment B | 10 | 128.5 | 88.96 (70.52, 112.22) |
| | Treatment A1 | 10 | 144.4 | |
| | Treatment D | 10 | 131.3 | 90.94 (73.41, 112.64) |
| | Treatment A1 | 10 | 144.4 | |
| C24 (ng/mL) | Treatment B | 10 | 4.007 | 106.51 (93.73, 121.03) |
| | Treatment A1 | 10 | 3.762 | |
| | Treatment D | 10 | 4.185 | 111.24 (98.88, 125.14) |
| | Treatment A1 | 10 | 3.762 | |

Treatment A1 150 mg AZD9977 (AZD9977 capsule 50 mg + AZD9977 capsule 100 mg) and 10 mg dapagliflozin tablet, fasted in Group 1. Treatment B 150 mg AZD9977 + 10 mg dapagliflozin Capsule 1, fasted. Treatment D 150 mg AZD9977 + 10 mg dapagliflozin Capsule 2, fasted. CI Confidence interval; LS Least-squares; N Number of subjects per treatment group. Geometric mean ratio and corresponding 90% CI are back-transformed.

Result based on a linear mixed effect analysis of variance (ANOVA) of log transformed PK parameter with sequence, period, treatment as fixed-effect and subject nested within sequence as random effect.

FIG. 7B

| Parameter (Units) | Treatment | N | Geometric LS Mean | Comparison of Treatment Groups |
|---|---|---|---|---|
| | | | | Geometric Mean Ratio (90% CI) |
| AUCinf (h*ng/mL) | Treatment C | 10 | 536.0 | 103.65 (99.92, 107.52) |
| | Treatment B | 10 | 517.2 | |
| | Treatment E | 10 | 523.4 | 98.47 (94.31, 102.82) |
| | Treatment D | 10 | 531.5 | |
| AUClast (h*ng/mL) | Treatment C | 10 | 527.0 | 104.19 (100.51, 108.00) |
| | Treatment B | 10 | 505.9 | |
| | Treatment E | 10 | 514.6 | 98.36 (94.28, 102.62) |
| | Treatment D | 10 | 523.2 | |
| Cmax (ng/mL) | Treatment C | 10 | 84.51 | 65.78 (53.16, 81.39) |
| | Treatment B | 10 | 128.5 | |
| | Treatment E | 10 | 94.73 | 72.12 (56.11, 92.71) |
| | Treatment D | 10 | 131.3 | |
| C24 (ng/mL) | Treatment C | 10 | 4.487 | 111.96 (99.58, 125.89) |
| | Treatment B | 10 | 4.007 | |
| | Treatment E | 10 | 4.242 | 101.36 (88.28, 116.38) |
| | Treatment D | 10 | 4.185 | |

Treatment B 150 mg AZD9977 + 10 mg dapagliflozin Capsule 1, fasted. Treatment C 150 mg AZD9977 + 10 mg dapagliflozin Capsule 1, fed. Treatment D 150 mg AZD9977 + 10 mg dapagliflozin Capsule 2, fasted. Treatment E 150 mg AZD9977 + 10 mg dapagliflozin Capsule 2, fed. CI Confidence interval; LS Least-squares; N Number of subjects per treatment group. Geometric mean ratio and corresponding 90% CI are back-transformed.
Result based on a linear mixed effect analysis of variance (ANOVA) of log transformed PK parameter with sequence, period, treatment as fixed-effect and subject nested within sequence as random effect.

FIG. 8A

| Parameter (Units) | Treatment | N | Geometric LS Mean | Comparison of Treatment Groups |
|---|---|---|---|---|
| | | | | Geometric Mean Ratio (90% CI) |
| | Treatment F | 10 | 512.5 | 102.73 (97.82, 107.89) |
| | Treatment A2 | 10 | 498.8 | |
| AUCinf (h*ng/mL) | Treatment G | 10 | 542.5 | 108.76 (103.53, 114.25) |
| | Treatment A2 | 10 | 498.8 | |
| | Treatment H | 10 | 528.4 | 105.93 (100.86, 111.25) |
| | Treatment A2 | 10 | 498.8 | |
| | Treatment F | 10 | 503.0 | 103.05 (98.05, 108.31) |
| | Treatment A2 | 10 | 488.1 | |
| AUClast (h*ng/mL) | Treatment G | 10 | 528.0 | 108.17 (102.90, 113.72) |
| | Treatment A2 | 10 | 488.1 | |
| | Treatment H | 10 | 520.9 | 106.71 (101.53, 112.15) |
| | Treatment A2 | 10 | 488.1 | |
| | Treatment F | 10 | 122.4 | 77.78 (64.90, 93.22) |
| | Treatment A2 | 10 | 157.4 | |
| Cmax (ng/mL) | Treatment G | 10 | 152.1 | 96.64 (80.56, 115.93) |
| | Treatment A2 | 10 | 157.4 | |
| | Treatment H | 10 | 116.1 | 73.76 (61.54, 88.40) |
| | Treatment A2 | 10 | 157.4 | |
| | Treatment F | 10 | 3.608 | 107.49 (97.02, 119.08) |
| | Treatment A2 | 10 | 3.356 | |
| C24 (ng/mL) | Treatment G | 10 | 3.741 | 111.47 (100.57, 123.56) |
| | Treatment A2 | 10 | 3.356 | |
| | Treatment H | 10 | 3.855 | 114.85 (103.67, 127.24) |
| | Treatment A2 | 10 | 3.356 | |

Treatment A2 150 mg AZD9977 (AZD9977 capsule 50 mg + AZD9977 capsule 100 mg) and 10 mg dapagliflozin tablet, fasted in Group 2. Treatment F 150 mg AZD9977 + 10 mg dapagliflozin Capsule 3, fasted. Treatment G 150 mg AZD9977 + 10 mg dapagliflozin Capsule 4, fasted. Treatment H 10 mg dapagliflozin capsule, fasted. CI Confidence interval; LS Least-squares; N Number of subjects per treatment group. Geometric mean ratio and corresponding 90% CI are back-transformed.
Result based on a linear mixed effect analysis of variance (ANOVA) of log transformed PK parameter with sequence, period, treatment as fixed-effect and subject nested within sequence as random effect.

FIG. 8B

| Parameter (Units) | Treatment | N | Geometric LS Mean | Comparison of Treatment Groups |
|---|---|---|---|---|
| | | | | Geometric Mean Ratio (90% CI) |
| AUCinf (h*ng/mL) | Treatment B | 10 | 517.9 | 93.59 (88.63, 98.82) |
| | Treatment G | 10 | 553.4 | |
| | Treatment D | 10 | 533.1 | 101.69 (96.08, 107.62) |
| | Treatment F | 10 | 524.3 | |
| AUClast (h*ng/mL) | Treatment B | 10 | 505.1 | 93.66 (88.64, 98.95) |
| | Treatment G | 10 | 539.4 | |
| | Treatment D | 10 | 523.6 | 101.59 (95.4, 107.58) |
| | Treatment F | 10 | 515.4 | |
| Cmax (ng/mL) | Treatment B | 10 | 128.7 | 84.30 (69.52, 102.22) |
| | Treatment G | 10 | 152.7 | |
| | Treatment D | 10 | 133.3 | 107.37 (87.33, 132.03) |
| | Treatment F | 10 | 124.1 | |
| C24 (ng/mL) | Treatment B | 10 | 3.988 | 101.53 (89.73, 114.89) |
| | Treatment G | 10 | 3.927 | |
| | Treatment D | 10 | 4.101 | 108.09 (94.91, 123.09) |
| | Treatment F | 10 | 3.794 | |

Treatment B 150 mg AZD9977 + 10 mg dapagliflozin Capsule 1, fasted. Treatment D 150 mg AZD9977 + 10 mg dapagliflozin Capsule 2, fasted. Treatment F 150 mg AZD9977 + 10 mg dapagliflozin Capsule 3, fasted. Treatment G 150 mg AZD9977 + 10 mg dapagliflozin Capsule 4, fasted. CI Confidence interval; LS Least-squares; N Number of subjects per treatment group.
Result based on a linear mixed effect analysis of variance (ANOVA) of log transformed PK parameter with period, treatment as fixed-effect and subject nested within sequence as random effect and adjusting for the values of reference Treatment A. Geometric mean ratio and corresponding 90% CI are back-transformed.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7919598 B
- WO 2016001631 A


### Non-patent literature cited in the description


- **BRAUNWALD et al.** *Lancet*, 2015, vol. 385 (9970), 812-24
- **AMBROSY et al.** *Curr Heart Fail Rep.*, 2014, vol. 11 (4), 416-427
- **SARRAF et al.** *Clin JAm Soc Nephrol.*, 2009, vol. 4 (12), 2013-26
- **ATHER et al.** *J Am Coll Cardiol.*, 2012, vol. 59 (11), 998-1005
- SGLT2 Inhibitors. National Kidney Foundation, June 2022
- **ABRAHAM, WT et al.** *JACC: Heart Failure*, 2020, vol. 8, 961-972
- Guidance for Industry - Food-Effect Bioavailability and Fed Bioequivalence Studies. U.S. Food and Drug Administration, Center for Drug Evaluation and Research (CDER), December 2002
- Guidance for Industry - Bioavailability and Bioequivalence Studies Submitted in NDAs or INDs-General Considerations - Draft Guidance. U.S. Food and Drug Administration, Center for Drug Evaluation and Research (CDER), March 2014